# EUROPEAN PATENT SPECIFICATION

(11) **EP 4 263 541 B1**
(45) Date of publication and mention of the grant of the patent: **15.07.2026**
(21) Application number: 21836543.5
(22) Date of filing: 16.12.2021
(51) Int. Cl.: C07D 471/04, A61P 35/00, A61K 31/519

(54) **NEW ARYL-PYRIDO-PYRIMIDIN-ONE DERIVATIVES**
NEUE ARYL-PYRIDO-PYRIMIDIN-ON-DERIVATE
NOUVEAUX DÉRIVÉS D'ARYL-PYRIDO-PYRIMIDIN-ONE

(30) Priority: 18.12.2020 EP 20215299
(43) Date of publication of application: 25.10.2023
(73) Proprietor: F. Hoffmann-La Roche AG, 4070 Basel (CH)
(72) Inventor: DOLENTE, Cosimo, 4070 Basel (CH); HEWINGS, David Stephen, 4070 Basel (CH); HUNZIKER, Daniel, 4070 Basel (CH); JUNG, Erik, 4070 Basel (CH); PETTAZZONI, Piergiorgio Francesco Tommaso, 4070 Basel (CH); RICKLIN, Fabienne, 4070 Basel (CH); RIEMER, Claus, 4070 Basel (CH); WICHMANN, Juergen, 4070 Basel (CH)
(74) Representative: Vitra, Hermeto
(86) International application number: PCT/EP2021/086050
(87) International publication number: WO 2022/129260

(56) References cited:
- WO-A1-2009/012283
- WO-A1-2012/118492
- LI REN ET AL: "The discovery of potent and selective pyridopyrimidin-7-one based inhibitors of B-Rafkinase", BIOORGANIC & MEDICINAL CHEMISTRY LETTERS, ELSEVIER, AMSTERDAM, NL, vol. 22, no. 10, 3 April 2012 (2012-04-03), pages 3387 - 3391, XP028479266, ISSN: 0960-894X, [retrieved on 20120410], DOI: 10.1016/J.BMCL.2012.04.015

## Description

The present invention relates to organic compounds useful for therapy and/or prophylaxis in a mamal, and in particular to compounds that modulate BRAF activity.

The invention relates in particular to a compound of formula (I) 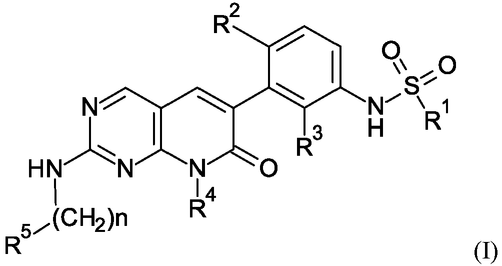 wherein
R¹ is dialkylamino, cycloalkyl(alkylamino), haloheterocycloalkyl, heterocycloalkyl, or cycloalkyl;
R² and R³ are independently selected from hydrogen, cyano and halogen;
R⁴ is alkyl, alkoxycarbonylheterocycloalkyl, aminoalkoxyalkoxyalkyl, heterocycloalkyl, alkylcarbonylaminoalkoxyalkoxyalkyl, alkylcarbonylheterocycloalkyl, alkylcarbonylaminocycloalkyl or phenylalkoxyalkyl; and
n = 0, 1, 2, 3, 4 or 5; and
R⁵ is alkoxycarbonyl, alkoxycarbonylalkylphenyl, alkoxycarbonylalkylheterocycloalkyl, alkoxycarbonylheterocycloalkyl, alkylamino, alkylcarbonyl(alkylamino), alkylcarbonyl(alkylamino)alkylphenyl, alkylcarbonylamino, alkylcarbonylaminoalkoxy, alkylcarbonylaminoalkoxyalkoxy, alkylcarbonylheterocycloalkylaminocarbonyl, alkylcarbonylaminoalkylphenyl, alkylcarbonyl(hydroxyheterocycloalkyl), alkylcarbonylheterocycloalkyl, alkylcarbonylaminocycloalkyl, aminoalkoxy, cycloalkyl, heterocycloalkyl, heterocycloalkylaminocarbonyl, phenyloxy, hydroxyalkoxyalkoxy, phenyl or hydrogen;
or a pharmaceutically acceptable salt thereof.

The Rapidly Accelerated Fibrosarcoma (RAF) class of serine-threonine kinases comprise three members (ARAF, BRAF, RAF1) that compose the first node of the MAP kinase signalling pathway. Despite the apparent redundancy of the three RAF isoforms in signalling propagation through phosphorylation of MEK1 and 2, frequent oncogenic activating mutations are commonly found only for BRAF. In particular, substitution of V600 with glutamic acid or lysine renders the kinase highly activated with consequent hyper-stimulation of the MAPK pathway, independently from external stimulations (Cell. 2015 Jun 18; 161(7): 1681-1696.)

Mutant BRAF is a targetable oncogenic driver and three BRAF inhibitors (vemurafenib, dabrafenib and encorafenib) reached the market up to now showing efficacy in BRAFV600E-positive melanoma. However, rapid acquisition of drug resistance is almost universally observed and the duration of the therapeutic benefits for the targeted therapy remains limited.

Moreover, the developed BRAF inhibitors revealed an unexpected and "paradoxical" ability to repress MAPK signalling in BRAFV600E-driven tumours while the same inhibitors presented MAPK stimulatory activities in BRAF wild type (WT) models (N Engl J Med 2012; 366:271-273; and British Journal of Cancer volume 111, pages640-645(2014)).

Mechanistic studies on the RAF paradox then clarified that oncogenic BRAFV600E phosphorylates MEK 1/2 in its monomeric cytosolic form while WT BRAF and RAF1 activation requires a complex step of events including cell membrane translocation and homo and/or heterodimerization promoted by activated RAS (KRAS, NRAS, HRAS) (Nature Reviews Cancer volume 14, pages455-467(2014)).

The binding of inhibitors like vemurafenib, dabrafenib or encorafenib to a WT BRAF or RAF1 protomer, quickly induces RAF homo and/or hetero dimerization and membrane association of the newly formed RAF dimer. In the dimeric conformation, one RAF protomer allosterically induces conformational changes of the second resulting in a kinase active status and, importantly, in a conformation unfavourable for the binding of the inhibitor. The dimer induced by drug treatment, as a result, promotes MEK phosphorylation by the catalysis operated by the unbound protomer with hyperactivation of the pathway.

The RAF paradox results in two clinically relevant consequences: 1) accelerated growth of secondary tumours upon BRAFi monotherapy (mainly keratochantoma and squamous-cell carcinomas) (N Engl J Med 2012; 366:271-273) and 2) the acquisition of drug resistance in the setting of BRAFi monotherapy as well as in combinations of BRAFi+MEKi presents activation of dimer-mediated RAF signalling by genetically driven events including RAS mutations, BRAF amplifications, expression of dimeric-acting BRAF splice variants (Nature Reviews Cancer volume 14, pages455-467(2014)).

The present invention relates to the surprising finding that the BRAF inhibitor of formula (I) shows considerably less paradoxial activation of the MAPK signalling pathway while retaining high potency. This compound can also be referred to as a paradox breaker or RAF paradox breaker, compared to compounds inducing the RAF paradox (and which could be referred to as paradox inducers or RAF paradox inducers).

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain C1-C8 alkyl groups are methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl, pentyl and hexyl. Methyl, ethyl, propyl and pentyl are particular examples of "alkyl" in the compound of formula (I).

The term "cycloalkyl", alone or in combination, signifies a cycloalkyl ring with 3 to 8 carbon atoms and particularly a cycloalkyl ring with 3 to 6 carbon atoms. Examples of "cycloalkyl" are cyclopropyl, cyclobutyl, cyclopentyl, cyclohexyl, cycloheptyl and cyclooctanyl. Particular examples of "cycloalkyl" are cyclopropyl, cyclobutyl and cyclopentyl.

The term "alkoxy" or "alkyloxy", alone or in combination, signifies a group of the formula alkyl-O- in which the term "alkyl" has the previously given significance, such as methoxy, ethoxy, n-propoxy, isopropoxy, n-butoxy, isobutoxy, sec-butoxy and tert.-butoxy. Particular examples of "alkoxy" are methoxy and ethoxy.

The term "oxy", alone or in combination, signifies the -O- group.

The term "oxo", alone or in combination, signifies the =O group.

The terms "halogen" or "halo", alone or in combination, signifies fluorine, chlorine, bromine or iodine and particularly fluorine, chlorine or bromine, more particularly fluorine. The term "halo", in combination with another group, denotes the substitution of said group with at least one halogen, particularly substituted with one to five halogens, particularly one to four halogens, i.e. one, two, three or four halogens.

The term "alkylamino", alone or in combination, signifies an alkyl group linked to a - NH- group. The term "dialkylamino", alone or in combination, signifies two alkyl groups linked to a -N- atom.

The terms "hydroxyl" and "hydroxy", alone or in combination, signify the -OH group.

The term "carbonyl", alone or in combination, signifies the -C(O)- group.

The term "amino", alone or in combination, signifies the primary amino group (-NH₂), the secondary amino group (-NH-), or the tertiary amino group (-N-).

The term "sulfonyl", alone or in combination, signifies the -SO₂- group.

The term "cyano", alone or in combination, signifies the -CN group.

The term "heterocycloalkyl" denotes a monovalent saturated or partly unsaturated mono- or bicyclic ring system of 4 to 9 ring atoms, comprising 1, 2, or 3 ring heteroatoms selected from N, O and S, the remaining ring atoms being carbon. Bicyclic means consisting of two cycles having one or two ring atoms in common. Examples of "heterocycloalkyl" are pyrrolidinyl, piperidinyl, 1,3-dihydroisoindolyl, 1H-isoquinolinyl, azetidinyl, and piperazinyl. Particular examples of "heterocycloalkyl" are pyrrolidinyl, piperidinyl and piperazinyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition these salts may be prepared form addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula (I) can also be present in the form of zwitterions. Particularly preferred pharmaceutically acceptable salts of the compound of formula (I) are the salts of trifluoroacetic acid, hydrochloric acid, hydrobromic acid, sulfuric acid, phosphoric acid and methanesulfonic acid.

If one of the starting materials or compounds of formula (I) contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compound of formula (I) can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

The term "asymmetric carbon atom" means a carbon atom with four different substituents. According to the Cahn-Ingold-Prelog Convention an asymmetric carbon atom can be of the "R" or "S" configuration.

The invention thus relates to:
A compound of formula (I) or a pharmaceutically acceptable thereof, wherein R¹ is dialkylamino, (cycloalkyl)(alkyl)amino, halopyrrolidinyl, pyrrolidinyl or cycloalkyl;
A compound of formula (I) or a pharmaceutically acceptable thereof, wherein R¹ is dialkylamino, (cycloalkyl)(alkyl)amino, halopyrrolidinyl or pyrrolidinyl;
A compound of formula (I) or a pharmaceutically acceptable thereof, wherein R¹ is cycloalkyl;
A compound of formula (I) or a pharmaceutically acceptable thereof, wherein R¹ is dialkylamino, (cycloalkyl)(alkyl)amino, fluoropyrrolidinyl, pyrrolidinyl or cycloalkyl;
A compound of formula (I) or a pharmaceutically acceptable thereof, wherein R¹ is (ethyl)(methyl)amino, (cyclopropyl)(methyl)amino, fluoropyrrolidinyl, pyrrolidinyl or cyclopentyl;
A compound of formula (I) or a pharmaceutically acceptable thereof, wherein R¹ is (ethyl)(methyl)amino, cyclopropyl(methylamino), fluoropyrrolidinyl or pyrrolidinyl;
A compound of formula (I) or a pharmaceutically acceptable thereof, wherein R¹ is cyclopentyl;
A compound of formula (I) or a pharmaceutically acceptable thereof, wherein R¹ is dialkylamino or haloheterocycloalkyl;
A compound of formula (I) or a pharmaceutically acceptable thereof, wherein R¹ is (ethyl)(methyl)amino or fluoropyrrolidinyl;
A compound of formula (I) or a pharmaceutically acceptable thereof, wherein R² and R³ are independently selected from hydrogen, cyano and fluorine;
A compound of formula (I) or a pharmaceutically acceptable thereof, wherein R² and R³ are both fluorine at the same time;
A compound of formula (I) or a pharmaceutically acceptable thereof, wherein R⁴ is methyl, tert-butyloxycarbonylpyrrolidinyl, aminoethoxyethoxyethyl, pyrrolidinyl, methylcarbonylaminoethoxyethoxyethyl, methylcarbonylpyrrolidinyl, methylcarbonylaminocyclobutyl or phenylmethoxyethyl;
A compound of formula (I) or a pharmaceutically acceptable thereof, wherein R⁴ is alkyl;
A compound of formula (I) or a pharmaceutically acceptable thereof, wherein R⁴ is methyl;
A compound of formula (I) or a pharmaceutically acceptable thereof, wherein n = 0, 1, 2, 3 or 5;
A compound of formula (I) or a pharmaceutically acceptable thereof, wherein n = 1, 2, 3 or 5;
A compound of formula (I) or a pharmaceutically acceptable thereof, wherein R⁵ is alkoxycarbonyl, alkoxycarbonylalkylphenyl, alkoxycarbonylalkylheterocycloalkyl, alkoxycarbonylheterocycloalkyl, alkylamino, alkylcarbonyl(alkylamino), alkylcarbonyl(alkylamino)alkylphenyl, alkylcarbonylamino, alkylcarbonylaminoalkoxy, alkylcarbonylaminoalkoxyalkoxy, alkylcarbonylheterocycloalkylaminocarbonyl, alkylcarbonylaminoalkylphenyl, alkylcarbonyl(hydroxyheterocycloalkyl), alkylcarbonylheterocycloalkyl, alkylcarbonylaminocycloalkyl, aminoalkoxy, cycloalkyl, heterocycloalkyl, heterocycloalkylaminocarbonyl, phenyloxy, hydroxyalkoxyalkoxy, phenyl or hydrogen;
A compound of formula (I) or a pharmaceutically acceptable thereof, wherein R⁵ is tert-butyloxycarbonyl, tert-butyloxycarbonylmethylphenyl, methoxycarbonylmethylphenyl, tert-butyloxycarbonylpiperidinyl, tertbutyloxycarbonylpiperazinyl, methylamino, methylcarbonyl(methylamino), methylcarbonyl(methylamino)methylphenyl, methylcarbonylamino, methylcarbonylaminoethoxyethoxy, methylcarbonylpiperidinylaminocarbonyl, methylcarbonylaminomethylphenyl, methylcarbonyl(hydroxypiperidinyl), methylcarbonylpiperidinyl, methylcarbonylpiperazinyl, methylcarbonylpiperidinyl, methylcarbonyl-1,3-dihydroisoindolyl, methylcarbonyl-1H-isoquinolinyl, aminoethoxy, cyclopropyl, cyclopentyl, piperidinyl, piperidinylaminocarbonyl, phenyloxy, hydroxyethoxyethoxy, phenyl or hydrogen;
A compound of formula (I) or a pharmaceutically acceptable thereof, wherein R⁵ is phenyl, alkoxycarbonyl, cycloalkyl, alkylcarbonylpiperidinyl, alkylcarbonylpiperazinyl, alkylcarbonylamino, alkylcarbonyl(alkylamino), alkylcarbonylaminoalkylphenyl or alkylcarbonyl(alkylamino)alkylphenyl; and
A compound of formula (I) or a pharmaceutically acceptable thereof, wherein R⁵ is phenyl, tert-butyloxycarbonyl, cyclopropyl, methylcarbonylpiperidinyl, methylcarbonylpiperazinyl, methylcarbonylamino, methylcarbonyl(methylamino), methylcarbonylaminomethylphenyl or methylcarbonyl(methylamino)methylphenyl.

The invention further relates to a compound of formula (I) selected from
tert-butyl 4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]piperidine-1-carboxylate;
(3R)-N-[2,4-difluoro-3-[8-methyl-7-oxo-2-(2-phenylethylamino)pyrido[2,3-d]pyrimidin-6-yl]phenyl]-3-fluoropyrrolidine-1-sulfonamide;
tert-butyl 4-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]butanoate;
(3R)-N-[2,4-difluoro-3-[2-[2-[2-(2-hydroxyethoxy)ethoxy]ethylamino]-8-methyl-7-oxopyrido[2,3-d]pyrimidin-6-yl]phenyl]-3-fluoropyrrolidine-1-sulfonamide;
(3R)-N-[2,4-difluoro-3-[8-methyl-7-oxo-2-(2-phenoxyethylamino)pyrido[2,3-d]pyrimidin-6-yl]phenyl]-3-fluoropyrrolidine-1-sulfonamide;
(3R)-N-[3-[2-(cyclopentylamino)-8-methyl-7-oxopyrido[2,3-d]pyrimidin-6-yl]-2,4-difluorophenyl]-3-fluoropyrrolidine-1-sulfonamide;
tert-butyl 4-[2-[[6-[2,6-difluoro-3-(pyrrolidin-1-ylsulfonylamino)phenyl]-8-methyl-7-oxopyrido[2,3-d]pyrimidin-2-yl]amino]ethyl]piperidine-1-carboxylate;
6-[3-[[ethyl(methyl)sulfamoyl]amino]-2,6-difluorophenyl]-2-[2-[2-(2-hydroxyethoxy)ethoxy]ethylamino]-8-methyl-7-oxopyrido[2,3-d]pyrimidine;
(3R)-N-[3-[2-(cyclopropylmethylamino)-8-methyl-7-oxopyrido[2,3-d]pyrimidin-6-yl]-2,4-difluorophenyl]-3-fluoropyrrolidine-1-sulfonamide;
2-(cyclopropylmethylamino)-6-[3-[[ethyl(methyl)sulfamoyl]amino]-2,6-difluorophenyl]-8-methyl-7-oxopyrido[2,3-d]pyrimidine;
2-(cyclopropylmethylamino)-6-[3-[[cyclopropyl(methyl)sulfamoyl]amino]-2,6-difluorophenyl]-8-methyl-7-oxopyrido[2,3-d]pyrimidine;
tert-butyl 4-[2-[[6-[3-(cyclopentylsulfonylamino)-2,6-difluorophenyl]-8-methyl-7-oxopyrido[2,3-d]pyrimidin-2-yl]amino]ethyl]piperidine-1-carboxylate;
(3R)-N-[2,4-difluoro-3-[8-methyl-7-oxo-2-(2-piperidin-4-ylethylamino)pyrido[2,3-d]pyrimidin-6-yl]phenyl]-3-fluoropyrrolidine-1-sulfonamide hydrochloride;
(3R)-N-[3-[2-[2-(2-aminoethoxy)ethylamino]-8-methyl-7-oxopyrido[2,3-d]pyrimidin-6-yl]-2,4-difluorophenyl]-3-fluoropyrrolidine-1-sulfonamide hydrochloride;
(3R)-N-[2,4-difluoro-3-[8-methyl-2-[3-(methylamino)propylamino]-7-oxopyrido[2,3-d]pyrimidin-6-yl]phenyl]-3-fluoropyrrolidine-1-sulfonamide hydrochloride;
(3R)-N-[2,4-difluoro-3-[8-methyl-7-oxo-2-(3-piperidin-4-ylpropylamino)pyrido[2,3-d]pyrimidin-6-yl]phenyl]-3-fluoropyrrolidine-1-sulfonamide hydrochloride;
(3R)-N-[3-[2-[2-(1-acetylpiperidin-4-yl)ethylamino]-8-methyl-7-oxopyrido[2,3-d]pyrimidin-6-yl]-2,4-difluorophenyl]-3-fluoropyrrolidine-1-sulfonamide;
(3R)-N-[3-[2-[(1-acetylpiperidin-4-yl)methylamino]-8-methyl-7-oxopyrido[2,3-d]pyrimidin-6-yl]-2,4-difluorophenyl]-3-fluoropyrrolidine-1-sulfonamide;
(3R)-N-[3-[2-[(1-acetylazetidin-3-yl)amino]-8-methyl-7-oxopyrido[2,3-d]pyrimidin-6-yl]-2,4-difluorophenyl]-3-fluoropyrrolidine-1-sulfonamide;
N-[(1R,3R)-3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]cyclopentyl]acetamide;
N-[(1S,3S)-3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]cyclopentyl]acetamide;
N-[5-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]pentyl]acetamide;
(3R)-N-[3-[2-[2-(1-acetyl-4-hydroxypiperidin-4-yl)ethylamino]-8-methyl-7-oxopyrido[2,3-d]pyrimidin-6-yl]-2,4-difluorophenyl]-3-fluoropyrrolidine-1-sulfonamide;
N-[3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]propyl]-N-methylacetamide;
(3R)-N-[3-[2-[3-(1-acetylpiperidin-4-yl)propylamino]-8-methyl-7-oxopyrido[2,3-d]pyrimidin-6-yl]-2,4-difluorophenyl]-3-fluoropyrrolidine-1-sulfonamide;
N-[2-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethoxy]ethyl]acetamide;
N-[3-[[6-[3-[[ethyl(methyl)sulfamoyl]amino]-2,6-difluorophenyl]-8-methyl-7-oxopyrido[2,3-d]pyrimidin-2-yl]amino]propyl]-N-methylacetamide;
(3R)-N-[3-[2-[2-(4-acetylpiperazin-1-yl)ethylamino]-8-methyl-7-oxopyrido[2,3-d]pyrimidin-6-yl]-2,4-difluorophenyl]-3-fluoropyrrolidine-1-sulfonamide;
N-[2-[2-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethoxy]ethoxy]ethyl]acetamide;
N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide;
N-[[4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide;
N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]-N-methylacetamide;
2-[2-(1-acetylpiperidin-4-yl)ethylamino]-6-[2-cyano-3-[[ethyl(methyl)sulfamoyl]amino]phenyl]-8-methyl-7-oxopyrido[2,3-d]pyrimidine;
tert-butyl (3RS)-3-[2-(cyclopropylmethylamino)-6-[2,6-difluoro-3-[[(3R)-3-fluoropyrrolidin-1-yl]sulfonylamino]phenyl]-7-oxopyrido[2,3-d]pyrimidin-8-yl]pyrrolidine-1-carboxylate;
N-[2-[2-[2-[2-(cyclopropylmethylamino)-6-[2,6-difluoro-3-[[(3R)-3-fluoropyrrolidin-1-yl]sulfonylamino]phenyl]-7-oxopyrido[2,3-d]pyrimidin-8-yl]ethoxy]ethoxy]ethyl]acetamide;
(3R)-N-[3-[8-((3RS)-1-acetylpyrrolidin-3-yl)-2-(cyclopropylmethylamino)-7-oxopyrido[2,3-d]pyrimidin-6-yl]-2,4-difluorophenyl]-3-fluoropyrrolidine-1-sulfonamide;
tert-butyl 4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]piperazine-1-carboxylate;
tert-butyl 2-[4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]acetate;
methyl 2-[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]acetate;
tert-butyl 2-[5-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]pyrimidin-2-yl]acetate;
(3R)-N-[3-[2-[2-(2-acetyl-1,3-dihydroisoindol-5-yl)ethylamino]-8-methyl-7-oxopyrido[2,3-d]pyrimidin-6-yl]-2,4-difluorophenyl]-3-fluoropyrrolidine-1-sulfonamide; and
(3R)-N-[3-[2-[2-(2-acetyl-3,4-dihydro-1H-isoquinolin-7-yl)ethylamino]-8-methyl-7-oxopyrido[2,3-d]pyrimidin-6-yl]-2,4-difluorophenyl]-3-fluoropyrrolidine-1-sulfonamide;
or a pharmaceutically acceptable salt thereof.

The invention further relates to a compound of formula (I) selected from
(3R)-N-[2,4-difluoro-3-[8-methyl-7-oxo-2-(2-phenylethylamino)pyrido[2,3-d]pyrimidin-6-yl]phenyl]-3-fluoropyrrolidine-1-sulfonamide;
tert-butyl 4-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]butanoate;
(3R)-N-[3-[2-(cyclopropylmethylamino)-8-methyl-7-oxopyrido[2,3-d]pyrimidin-6-yl]-2,4-difluorophenyl]-3-fluoropyrrolidine-1-sulfonamide;
2-(cyclopropylmethylamino)-6-[3-[[ethyl(methyl)sulfamoyl]amino]-2,6-difluorophenyl]-8-methyl-7-oxopyrido[2,3-d]pyrimidine;
(3R)-N-[3-[2-[2-(1-acetylpiperidin-4-yl)ethylamino]-8-methyl-7-oxopyrido[2,3-d]pyrimidin-6-yl]-2,4-difluorophenyl]-3-fluoropyrrolidine-1-sulfonamide;
N-[5-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]pentyl]acetamide;
N-[3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]propyl]-N-methylacetamide;
(3R)-N-[3-[2-[3-(1-acetylpiperidin-4-yl)propylamino]-8-methyl-7-oxopyrido[2,3-d]pyrimidin-6-yl]-2,4-difluorophenyl]-3-fluoropyrrolidine-1-sulfonamide;
(3R)-N-[3-[2-[2-(4-acetylpiperazin-1-yl)ethylamino]-8-methyl-7-oxopyrido[2,3-d]pyrimidin-6-yl]-2,4-difluorophenyl]-3-fluoropyrrolidine-1-sulfonamide;
N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide;
N-[[4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide;
N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]-N-methylacetamide; and
2-[2-(1-acetylpiperidin-4-yl)ethylamino]-6-[2-cyano-3-[[ethyl(methyl)sulfamoyl]amino]phenyl]-8-methyl-7-oxopyrido[2,3-d]pyrimidine;
or a pharmaceutically acceptable salt thereof.

The preparation of compounds of formula (I) of the present invention may be carried out in sequential or convergent synthetic routes. Syntheses of the invention are shown in the following general schemes. The skills required for carrying out the reactions and purifications of the resulting products are known to those persons familiar with the art. In case a mixture of enantiomers or diastereoisomers is produced during a reaction, these enantiomers or diastereoisomers can be separated by methods described herein or known to those skilled in the art such as e.g. (chiral) chromatography or crystallization. The substituents and indices used in the following description of the processes have the significance given herein.

A general description of the invention is given in the following sections. Some of the general approaches are described in the literature [Ren et al., Bioorg. Med. Chem. Lett. 22 (2012), 3387]. To obtain compounds of formula (I), an appropriately substituted pyrimidine derivative of formula **A,** where Xa is a halogen such as Cl, Br or I can be treated with an amine H₂N-L-R⁵ in the presence of a base such as NEt₃, DIPEA, pyridine, Na₂CO₃, K₂CO₃, Cs₂CO₃ or the like in an appropriate solvent such as DMF, CH₃CN or similar to provide aminopyrimidine **B,** as shown in scheme 1. To obtain an aldehyde intermediate of formula **D,** several ways are possible. As outlined below in scheme 1, ester **B** can be reduced to alcohol **C** with a variety of reducing agents such as LiAlH₄, DIBAL, SMEAH (Red-Al) or the like in solvents such as THF, ether, DCM or similar at temperatures ranging from -78 to the boiling point of the solvent. Oxidation to aldehyde **D** can then be achieved by treatment with a variety of oxidizing agent such as MnO₂, Dess-Martin periodinane, pyridinium chlorochromate (PCC) or similar, in an appropriate solvent such as DCM, CHCl₃, THF, ether or the like at various temperatures. Alternatively, aldehyde **D** might be accessible by direct reduction of ester **B** with appropriate reducing agents such as DIBAL in an appropriate solvent such as THF, DCM or the like at low temperatures ranging from -78°C to 0 °C.

To obtain compounds with the aryl-pyrido-pyrimidinone core **F,** where the aryl moiety is carrying an amino group and the reactants can carry different substituents R², R³ and R⁴. an aldehyde **D** and aryl-acetic acid esters of formula **E** can be treated with a base such as potassium carbonate, cesium carbonate or the like in a solvent such as DMF, THF, CH₃CN or the like, at temperatures ranging from rt to the boiling point of the solvent. To obtain the sulfonylderivatives of formula **G,** intermediate **F** can be treated with a sulfonylchloride or sulfamoylchloride of formula **L,** where R¹ is either (branched) lower alkyl or a secondary amine substituted by lower alkyl/cycloalkyl, in the presence of a base such as NEt₃, DIPEA, K₂CO₃, Cs₂CO₃ or the like in solvents such as DCM, THF, CHCl₃ DMF, CH₃CN or similar or in pyridine optionally with or without DMAP.

Alternatively, to obtain an intermediate of formula I where the aryl moiety carries a substituent Xb suitable for Pd catalyzed reactions such as Cl, Br, I or OTf, an ester of formula **H** can be used as a starting material and similar reactions conditions as described for the transformation of **E** to **F** can be applied.

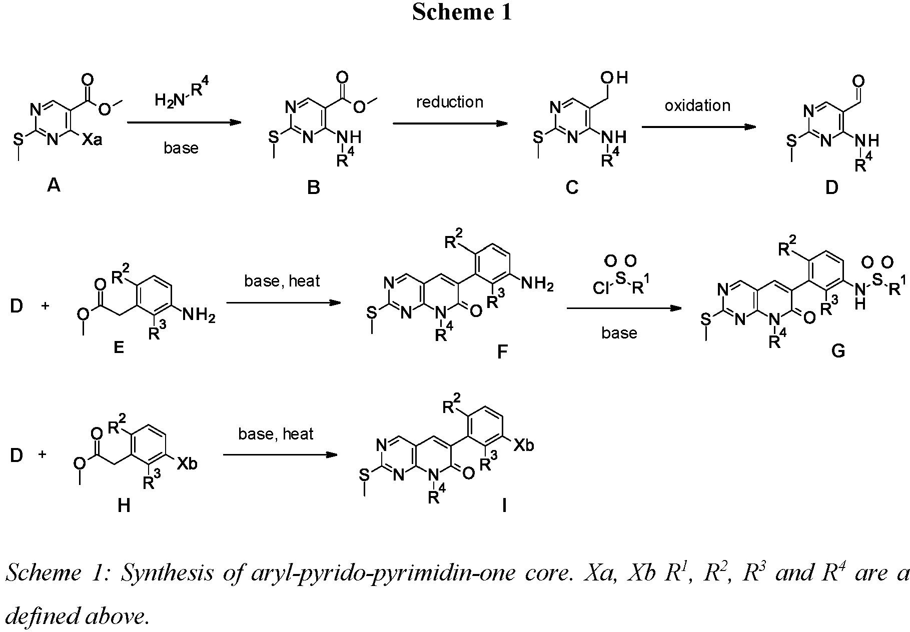

Introduction of the amine side chain R₅-L-NH₂ can be achieved in the same way for both intermediates **G** and **I** (see scheme 2). In a first step, Intermediates **G** or **I** are treated with an oxidizing agent such as mCPBA, H₂O₂, oxone or similar, in an appropriate solvent such as DCM, CHCl₃, water, MeOH, THF or the like at various temperatures to obtain methylsulfonyl derivatives of formula **L** or **N.** In a second step, the methylsulfonyl group can be displaced by treatment of either **L** or **N** with the appropriate amine R₅-L-NH₂ in a solvent such as ether, dioxane, THF, DCM, DMF CH₃CN or the like at various temperatures ranging from -20 °C to the boiling point of the solvent. Optionally, a base such as NEt₃, DIPEA, K₂CO₃, Cs₂CO₃ or similar can be present in this reaction, which will provide the compounds of formula **M** or **O.**

In case of intermediate **O,** introduction of the desired sulfonylamino- or sulfamoylamino group to provide intermediates or examples of formula **Q** can be achieved by applying for example a Buchwald type protocol in the presence of sulfonamid or sulfamoylamide **P** and a suitable catalyst such as Pd₂(dba)₃ and a ligand such as 2-dicyclohexylphosphino-2'-(N,N-dimethylamino)biphenyl in an appropriate solvent such as dioxane, DMF, toluene or similar, optionally in the presence of a base such as NaO^{tert}Bu, Cs₂CO₃, K₂CO₃, Na₂CO₃ or similar at temperatures ranging from rt to the boiling point of the solvent either with or without microwave irradiation. Many other combinations of catalysts, ligands, bases and solvents are possible based on analogous procedures reported in the literature for other transformations.

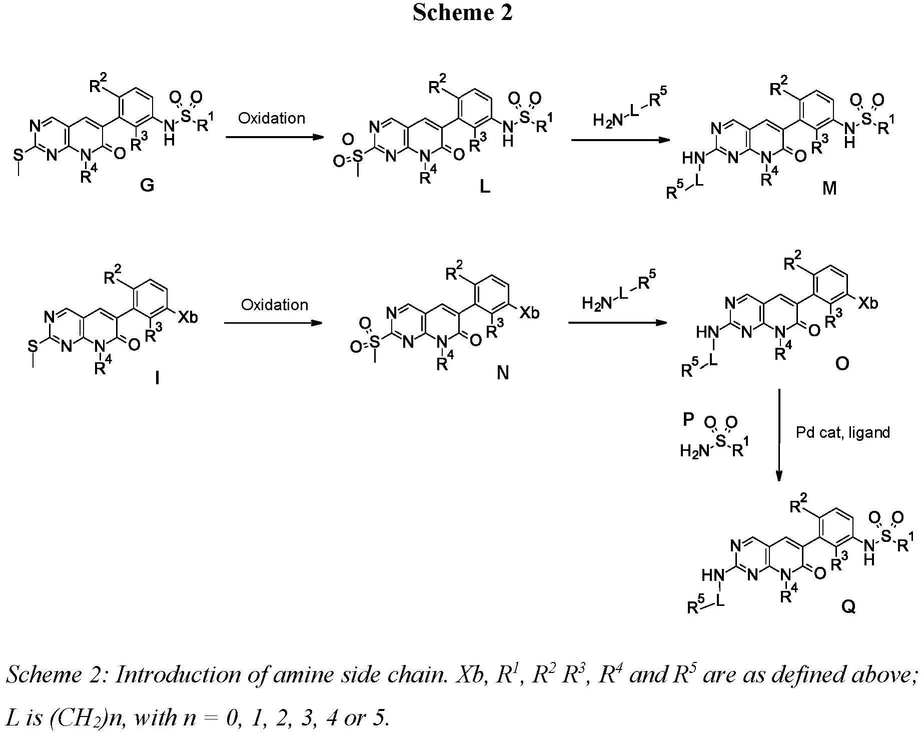

Depending on the nature of the substituents present in both R⁴ and R⁵ of intermediates and examples of formula **M** or **Q,** further functional group interchanges will be required to obtain subsequent intermediates or examples reported in this application.

If, for example, an intermediate or example carries a side chain R⁴ or R⁵ of the structure type **R** shown in scheme 3 with protected primary amine, then treatment with an acid HXc such as HCl, HBr, TFA or the like in a solvent such as water, dioxane, DCM, CHCl₃ or similar will provide intermediates or examples of formula **S** with a free amine side chain. Depending on the work-up of the reaction and isolation of the material, the side chain can be in a salt form as shown in scheme 3, structure **S,** or alternatively as a free amine (not shown). Optionally, intermediates or examples with a side chain R⁴ or R⁵ represented by structure **S** can be treated with an activated carboxylic acid **T,** where Ra is for example lower alkyl and LG is a leaving group and represents an activated carboxylic acid such as a carboxylic acid halide, anhydride, imidazolide or an intermediate coming from activation of the carboxylic acid with a peptide coupling reagent such as for example HATU, optionally in the presence of a base such as NEt₃, DIPEA, NaHCO₃, Na₂CO₃ or similar, in solvents such as water, DCM, THF, DMF or the like depending on the reagents used, to provide examples of formula **U** with R⁴ or R⁵ carrying an acyl-amino group.

Alternatively, if an intermediate or example carries a side chain R⁴ or R⁵ of the structure type **V** shown in scheme 3 with a protected secondary amine, for example as part of ring, then an analogous sequence as described above for **R, S** and **U** can be applied to provide intermediates and examples of formula **W** and **X,** respectively.

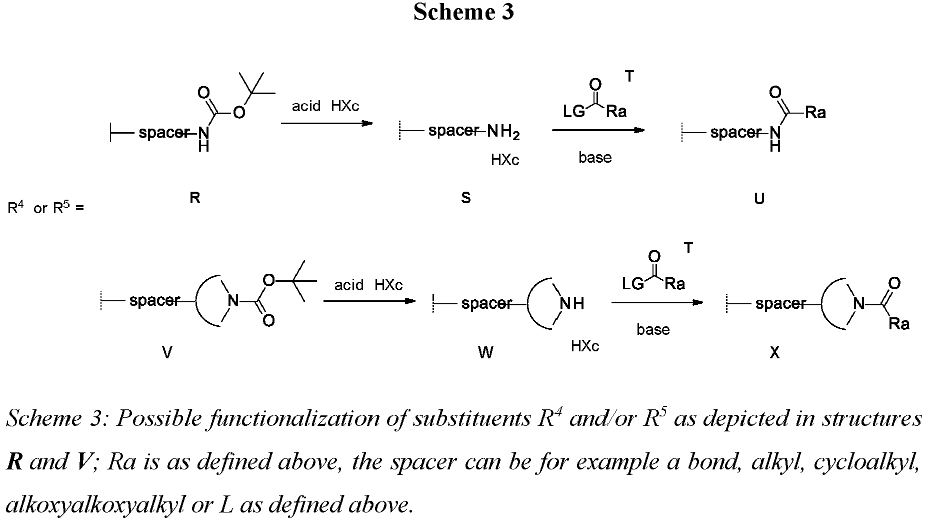

If, for example, an intermediate or example carries a side chain R⁵ of the structure type **Y** shown in scheme 4 with an ester group COORc, then, if for example Rb is ^{tert}Bu, treatment with an acid HXd such as HCl, HBr, TFA or the like in a solvent such as dioxane, DCM, CHCl₃ or similar will provide intermediates or examples of formula **Z** with a free carboxylic acid side chain. Alternatively, if Rb is for example methyl or ethyl or similar, a saponification protocol using NaOH, KOH or LiOH or similar in solvents such as water, MeOH, EtOH or THF or mixtures thereof at temperatures ranging from 0°C to the boiling point of the solvent can be applied to provide free carboxylic acid intermediates of formula Z. Intermediates Z can be further modified with primary (Rc = H) or secondary (Rc = e.g. alkyl) amines of formula AA using for example coupling reagents such as HATU or similar, optionally in the presence of a base such as NEt₃, DIPEA, NaHCO₃, Na₂CO₃ or the like, in solvents such as DCM, THF, DMF or the like, to provide amide intermediates of formula **AB.** If **AB** carries a BOC-protected amine, for example as part of a ring, an analogous deprotection-acylation sequence as described above in scheme 3 for **R, S** and **U** can be applied to provide intermediates and examples of formula **AC** and **AD.**

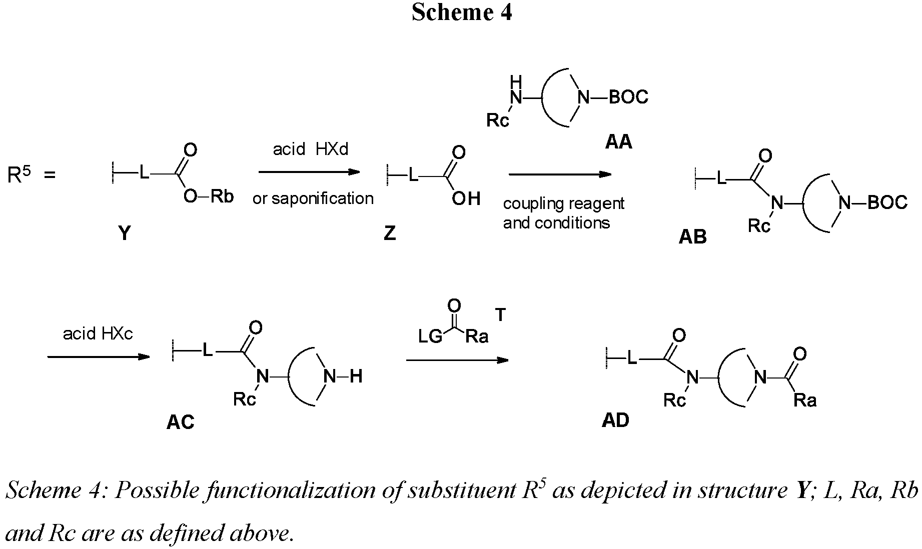

In some cases, where R⁵ incorporates a phenyl group substituted by cyano as shown in scheme 5, structure **AE** can be reduced to provide an aminomethyl derivative of formula **AF.** Conditions for this step are for example treatment with a reducing agent such as NaBH₄ or LiBH₄, optionally in the presence CoCl₂ or similar, or other reducing agents such as DIBAH. LiAlH₄, BH₃-Me₂S or the like, or by hydrogenation, in an appropriate solvent such as MeOH, EtOH, THF, ether or similar (depending on the reducing agent used) at temperatures ranging from -20°C to the boiling point of the solvent. As described previously in scheme 3 for example for the conversion of S to **U,** amine intermediate **AF** can be optionally acylated with reagents of formula **T** to provide acyl derivatives of formula **AG.**

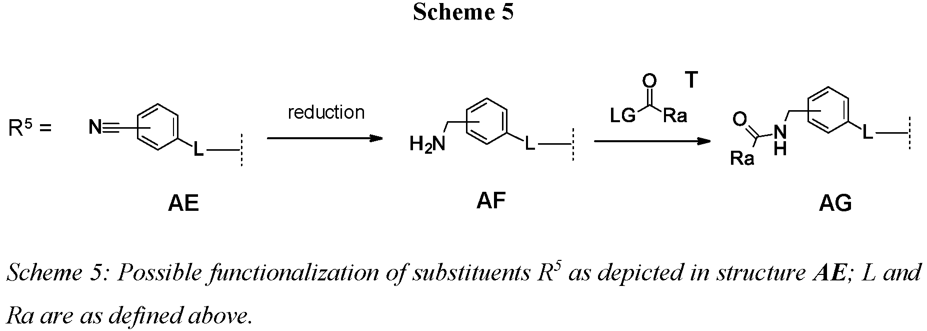

In some cases the starting material for the introduction of the desired side chains R⁵ in form of the amines R₅-L-NH₂ needed to be synthesized. The synthesis of such starting materials is outlined in scheme 6 below. In order to get access to arylethylamines of formula **AK,** where the aryl moiety is substituted with an alkylcarboxymethyl group in which Rd represents for example lower alkyl, an appropriately substituted phenylacetic acid ester **AH** can be treated with a suitably protected aminoethyltrifluoroborate reagent **AI,** where PG represents for example a compatible benzyloxycarbonyl group or similar, to provide intermediate **AJ.** The transformation is carried out in the presence of an appropriate Pd catalyst such as 1.1'-bis(diphenylphosphino)ferrocene-palladium(II)dichloride DCM complex or similar, in the presence of a base such as K₂CO₃, Cs₂CO₃ or KO^{tert}Bu or the like in a solvent such as water/toluene, water/dioxane, DMF or the like at temperatures ranging from rt to the boiling point of the solvent either with or without microwave irradiation. Deprotection of **AJ** to give amine **AK** can be achieved by catalytic hydrogenation with a suitable catalyst such as Pd/C or Pd(OH)₂ or similar in an appropriate solvent such as MeOH, EtOH or EtOAc, if PG is a benzyloxycarbonyl protecting group.

Similarly, suitably protected cyclic amine precursors **AN,** where PG* is an orthogonal protecting group as compared to PG and n is e.g. 1 or 2, can be made in an analogous way. The use of other catalysts such as for example methanesulfonato(2-dicyclohexylphosphino-2'-6'-di-i-propoxy-1,1'-biphenyl)(2-amino-1-1'biphenyl-2-yl)palladium(II) may be more appropriate for this transformation. The transformation from **AM** to **AN** can be carried out as describe above for **AJ** to **AK.**

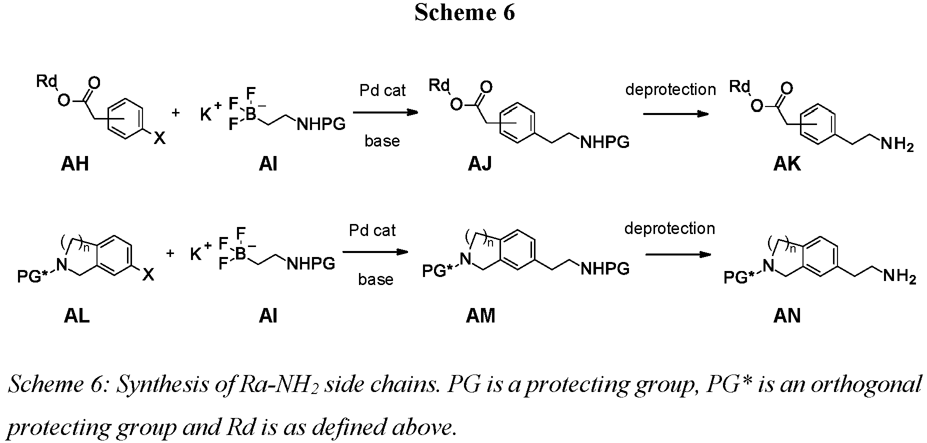

In some cases, an appropriate arylacetic acid ester intermediate H, where R³ is an electron withdrawing group such as CN, needed to be prepared. A possible approach is outlined in scheme 7. A commercially available, suitably substituted ortho-F-benzonitrile **AO** can be treated for example with diethylmalonate or similar in the presence of a base such as NaH, KO^{tert}Bu, LDA or the like in a solvent such as THF, ether or dioxane or similar at temperatures from -20°C to the boiling point of the solvent to afford intermediate **AP. AP** can be decarboxylated for example by treatment with LiCl in DMSO at elevated temperatures from 40 - 190 °C to provide arylacetic acid esters **AQ.**

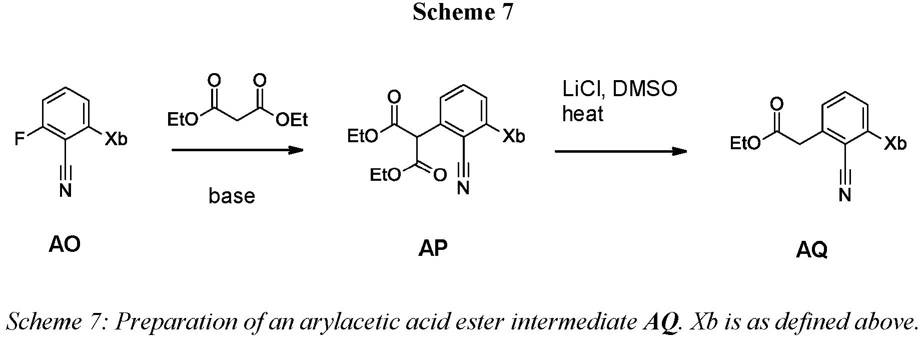

The invention thus also relates to a process for the preparation of a compound according to the invention, comprising one of the following steps:
(a) the reaction of a compound of formula (A1) 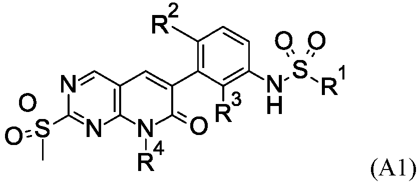
   with a compound of formula (A2) 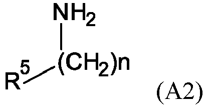
   in the presence of a base; or
(b) the reaction of a compound of formula (B1) 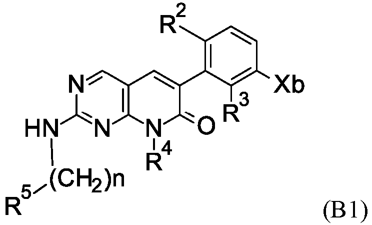
   with a compound of formula (B2) 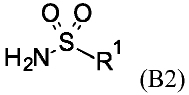
   in the presence of a base;
   wherein n, R¹, R², R³, R⁴ and R⁵ are as defined above.

The reaction of step (a) can conveniently be carried out in a solvent. The solvent can be for example DMF, THF, or a mixture thereof.

In the reaction of step (a) the base can be for example triethylamine or potassium carbonate. Conveniently the base is triethylamine.

Convenient conditions for the reaction of step (a) can be between around 0°C and around 50°C, particularly between around 5°C and around 30°C, more particularly between around 10°C and around 30°C.

Preferred conditions for the reaction of step (a) are the use of triethylamine in a DMF at around room temperature for between around 1-24 hrs, in particular between around 5-15 hrs.

The reaction of step (b) can conveniently be carried out in a solvent. The solvent can be for example dioxane, THF, or a mixture thereof.

In the reaction of step (b) the base can be for example cesium carbonate or potassium carbonate. Conveniently the base is cesium carbonate.

Convenient conditions for the reaction of step (b) can be between around 50°C and around 100°C, particularly between around 60°C and around 100°C, more particularly between around 70°C and around 80°C.

Preferred conditions for the reaction of step (b) are the use of cesium carbonate in dioxane at around 80°C for between around 10-24 hrs, in particular between around 15-18 hrs.

The invention also relates to a compound according to the invention when manufactured according to a process of the invention.

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula (I) may be formulated by mixing at ambient temperature at the appropriate pH, and at the desired degree of purity, with physiologically acceptable carriers, i.e., carriers that are nontoxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compound of formula (I) is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The invention also relates in particular to:
A compound of formula (I) or a pharmaceutically acceptable salt thereof, for use as therapeutically active substance;
A pharmaceutical composition comprising a compound of formula (I) or a pharmaceutically acceptable salt thereof, and a therapeutically inert carrier;
A compound of formula (I) or a pharmaceutically acceptable salt thereof, for use in the treatment or prophylaxis of cancer, in particular melanoma or NSCLC; and
A certain embodiment of the invention relates to the compound of formula (I) as described herein, or a pharmaceutically acceptable salt thereof, for the use in the treatment or prophylaxis of cancer, in particular melanoma or NSCLC, characterized by a BRAF mutation selected from V600E and V600K.

Furthermore, the invention includes all substituents in its corresponding deuterated form, wherever applicable, of the compound of formula (I).

Furthermore, the invention includes the corresponding carboxylic acid of the ester form, wherever applicable, of the compound of formula (I).

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

The invention will now be illustrated by the following examples which have no limiting character.

### Examples

### Abbreviations

BOC = tert-butyloxycarbonyl; CAS = chemical abstract service; dba = dibenzylideneacetone; DCM = dichloromethane; DIBAL = diisobutylaluminum hydride; DIPEA = diisopropylethylamine; DMF = dimethylformamide; DMA: = dimethylacetamide; DMAP = dimethylaminopyridine; DMSO = dimethyl sulfoxide; EA = ethyl acetate; ESI = electrospray ionization; EtOAc = ethyl acetate; Ex = example; HATU = hexafluorophosphate azabenzotriazole tetramethyl uronium; Int = intermediate; LC-MS = liquid chromatography coupled with mass spectrometry; LG = leaving group; mCPBA = meta chloroperbenzoic acid; MeOH = methanol; MS = mass spectrometry; NMR = nuclear magnetic resonance; PG = protecting group; rt = room temperature; SM = starting material; SMEAH = sodium bis(2-methoxyethoxy)aluminum hydride; TBME = tert-butyl methyl ether; THF = tetrahydrofuran; UV = ultraviolet.

### Intermediate IA1

### (3R)-N-[2,4-difluoro-3-(8-methyl-2-methylsulfonyl-7-oxopyrido[2,3-d]pyrimidin-6-yl)phenyl]-3-fluoropyrrolidine-1-sulfonamide

### Step 1: (3R)-N-[2,4-difluoro-3-(8-methyl-2-methylsulfanyl-7-oxopyrido[2,3-d]pyrimidin-6-yl)phenyl]-3-fluoropyrrolidine-1-sulfonamide

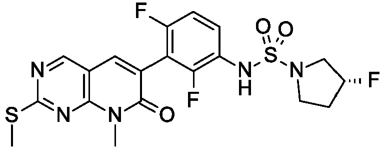

DCM (19.1 ml) was added to 6-(3-amino-2,6-difluorophenyl)-8-methyl-2-(methylthio)pyrido[2,3-d]pyrimidin-7(8H)-one (1.4 g, 4.19 mmol; CAS reg. No. 1377455-14-5, made according to Ren et al., Bioorg. Med. Chem. Lett. 22 (2012), 3387). At rt, pyridine (15.5 ml, 193 mmol), DMAP (26.1 mg, 209 µmol) and (R)-3-fluoropyrrolidine-1-sulfonyl chloride (CAS reg. No. 1411774-27-0; 1.57 g, 8.37 mmol) were added. The reaction mixture was then refluxed overnight at 60 °C. The mixture was diluted with 40 ml methyl-THF and saturated NaHCO₃ solution was added (40 ml) and the layers were separated. The aqueous layer was extracted with 3 x 40 ml methyl-THF. The combined organic layers were washed with brine (40 ml,) dried with anhydrous sodium sulfate, filtered and concentrated to give 2.96 g of a brown oil. The crude material was purified by flash chromatography by dissolving it in a minimal amount of DCM, applying it to an 80 g column, followed by elution with a gradient of 0-55% ethyl acetate in heptane. Fractions containing pure product were combined and concentrated *in vacuo* to give 1.33 g (66%) of the desired material as a pale yellow foam. MS (ESI): 486.0 [MH⁺].

### Step 2: (3R)-N-[2,4-difluoro-3-(8-methyl-2-methylsulfonyl-7-oxopyrido[2,3-d]pyrimidin-6-yl)phenyl]-3-fluoropyrrolidine-1-sulfonamide

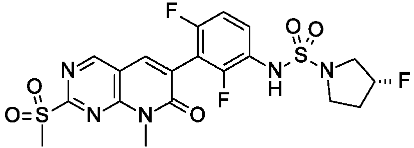

(3R)-N-(2,4-difluoro-3-(8-methyl-2-(methylthio)-7-oxo-7,8-dihydropyrido[2,3-d]pyrimidin-6-yl)phenyl)-3-fluoropyrrolidine-1-sulfonamide (1.33 g, 2.74 mmol) was dissolved in DCM (21.7 ml) and m-CPBA (1.18 g, 6.85 mmol) was added and the mixture was stirred at rt overnight. Analysis showed that some mono-oxidized product was still present and so more m-CPBA (473 mg, 2.74 mmol) was added and the reaction mixture was stirred for another hour. The reaction mixture was diluted with 40 ml DCM and 40 ml sat NaHCO₃ and the layers were separated. The aqueous layer was extracted with 3 x 40 ml DCM. The organic extracts were washed with 40 ml water, dried with anhydrous sodium sulfate, filtered and concentrated *in vacuo* to yield 1.52 g of a crude product. This material was dissolved in DCM/MeOH and was adsorbed to silica gel. The dried silica gel was applied to a precolumn fitted in front of a 80 g silica gel column. The column was eluted with a gradient of 0-70% ethyl acetate in heptane. Fractions containing pure product were combined and concentrated to give 1.15 g (81%) of the desired material as a pale yellow foam. MS (ESI): 518.2 [MH⁺].

### Intermediates IA2 - IA5

The following intermediates were made in analogy to intermediate IA1, steps 1 and 2, by using the appropriate reagents in reaction step 1:

| **Int.** | **Structure** | **Name** | **Reagents** | **MS (ESI⁺)** |
|---|---|---|---|---|
| IA2 | 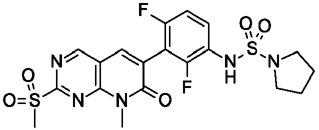 | N-[2,4-difluoro-3-(8-methyl-2-methylsulfonyl-7-oxopyrido[2,3-d]pyrimidin-6-yl)phenyl]pyrrolidi ne-1-sulfonamide | 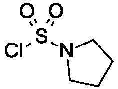 CAS Reg. No. 1689-02-7 | 500.2 [MH⁺] |
| IA3 | 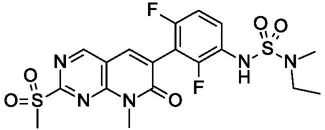 | 6-[3-[[ethyl(methyl)sulf amoyl]amino]-2,6-difluorophenyl]-8-methyl-2-methylsulfonyl-7-oxopyrido[2,3-d]pyrimidine | 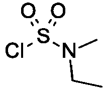 CAS Reg. No. 35856-61-2 | 488.2 [MH+] |
| IA4 | 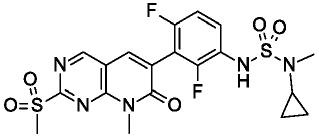 | 6-[3-[[cyclopropyl(meth yl)sulfamoyl]amino ]-2,6-difluorophenyl]-8-methyl-2-methylsulfonyl-7- | 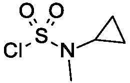 | 500.2 [MH+] |
| | | oxopyrido[2,3-d]pyrimidine | CAS Reg. No. 338449-00-6 | |
| IA5 | 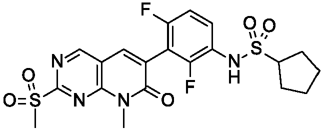 | N-[2,4-difluoro-3-(8-methyl-2-methylsulfonyl-7-oxopyrido[2,3-d]pyrimidin-6-yl)phenyl]cyclopen tanesulfonamide | 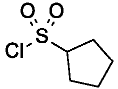 CAS Reg. No. 26394-17-2 | 499.2 [MH+] |
| | | | THF used instead of DCM as solvent in step 1. | |

### Intermediate IA6

### tert-Butyl 3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]azetidine-1-carboxylate

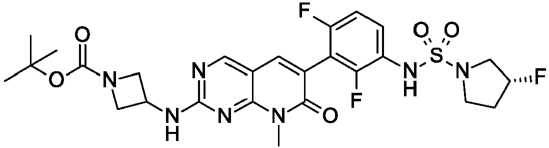

(3R)-N-(2,4-difluoro-3-(8-methyl-2-(methylsulfonyl)-7-oxo-7,8-dihydropyrido[2,3-d]pyrimidin-6-yl)phenyl)-3-fluoropyrrolidine-1-sulfonamide (intermediate IA1, 30 mg, 58 µmol) was dissolved in dioxane (386 µl) and tert-butyl 3-aminoazetidine-1-carboxylate (49.9 mg, 45.5 µl, 290 µmol) was added. The mixture was then allowed to stir for 1 hour at rt. LC-MS showed complete consumption of the starting material. The reaction mixture was concentrated *in vacuo* and was then dissolved in a minimal amount of DCM and applied to a 12 g silica gel column. The column was eluted with a gradient of 0-75% ethyl aceate in heptane. Product fractions were combined and concentrated *in vacuo* to give 28.2 mg (80%) of the desired material as a pale yellow crystalline solid. MS (ESI): 610.3 [MH⁺] .

### Intermediates IA7 - IA17

The following intermediates were made in analogy to intermediate IA6 by using the appropriate starting materials and reagents in the reaction:

| **Int.** | **Structure** | **Name** | **Reagents** | **MS (ESI)** |
|---|---|---|---|---|
| IA7 | 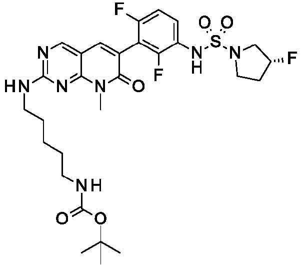 | tert-butyl N-[5-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]pentyl]carb amate | Intermediate IA1 and tert-butyl (5-aminopentyl) carbamate | 640.4 [MH⁺] |
| IA8 | 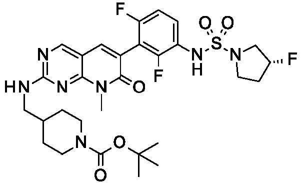 | tert-butyl 4-[[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]methyl]piperidine-1-carboxylate | Intermediate IA1 and tert-butyl 4-(aminomethy 1)piperidine-1-carboxylate | 650.5 [M-H]⁻ |
| IA9 | 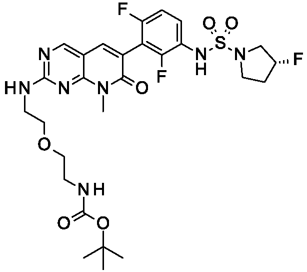 | tert-butyl N-[2-[2-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]ethoxy]eth yl]carbamate | Intermediate IA1 and tert-butyl (2-(2-aminoethoxy) ethyl)carbam ate | 642.2 [MH⁺] |
| IA10 | 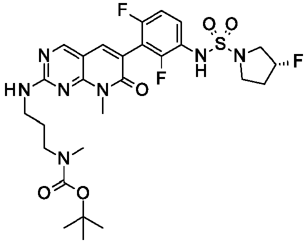 | tert-butyl N-[3-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]propyl]-N-methylcarbamate | Intermediate IA1 and tert-butyl (3-aminopropyl) (methyl)carb amate | 626.2 [MH⁺] |
| IA11 | 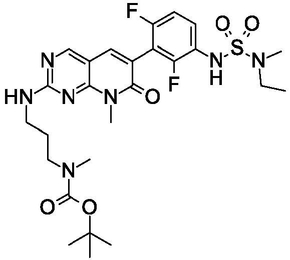 | tert-butyl N-[3-[[6-[3-[[ethyl(methyl)sulfa moyl]amino]-2,6-difluorophenyl]-8-methyl-7-oxopyrido[2,3-d]pyrimidin-2-yl]amino]propyl]-N-methylcarbamate | Intermediate IA3 and tert-butyl (3-aminopropyl) (methyl)carb amate | 596.3 [MH⁺] |
| IA12 | 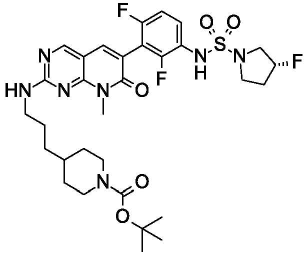 | tert-butyl 4-[3-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]propyl]pip eridine-1-carboxylate | Intermediate IA1 and tert-butyl 4-(3-aminopropyl) piperidine-1-carboxylate | 678.5 [M-H]⁻ |
| IA13 | 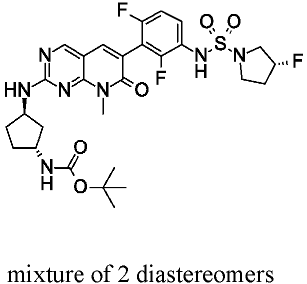 | tert-butyl N-[(1S,3S and 1R,3R)-3-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]cyclopenty l]carbamate | Intermediate IA1 and tert-butyl ((1S,3S and 1R,3R)-3-aminocyclop entyl)carbam ate | 638.3 [MH⁺] |
| IA14 | 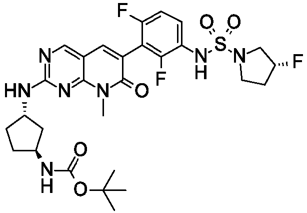 | tert-butyl N-[(1S,3S)-3-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]cyclopenty l]carbamate | Intermediate IA1 and tert-butyl ((1S,3S)-3-aminocyclopentyl)carbam ate | 638.3 [MH⁺] |
| IA15 | 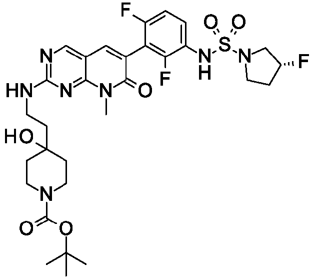 | tert-butyl 4-[2-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]ethyl]-4-hydroxypiperidine-1-carboxylate | Intermediate IA1 and tert-butyl 4-(2-aminoethyl)-4-hydroxypiper idine-1-carboxylate | 680.5 [MH⁺] |
| IA16 | 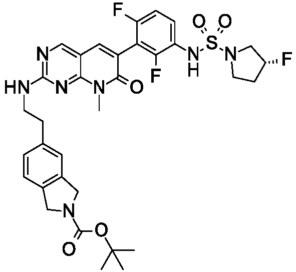 | tert-butyl 5-[2-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]ethyl]-1,3-dihydroisoindole-2-carboxylate | Intermediate IA1 and Intermediate IA34 | 700.5 [MH⁺] |
| IA17 | 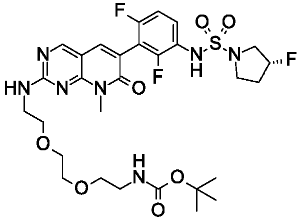 | tert-butyl N-[2-[2-[2-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]ethoxy]eth oxy]ethyl]carbamate | Intermediate IA1 and tert-butyl (2-(2-(2-aminoethoxy)ethoxy)ethyl) carbamate | 686.3 [MH⁺] |
| IA40 | 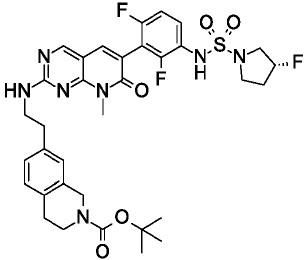 | tert-butyl 7-[2-[[6-[2,6-difluoro-3-[[(3R)-3-fluoropyrrolidin-1-yl]sulfonylamino]ph enyl]-8-methyl-7-oxopyrido[2,3-d]pyrimidin-2-yl]amino]ethyl]-3,4-dihydro-1H-isoquinoline-2-carboxylate | Intermediate IA1 and Intermediate IA39 Additional purification by prep. HPLC was required to obtain IA40 in adequate purity | 714.5 [MH⁺] |

### Intermediate IA18

### (3R)-N-[3-[2-(Azetidin-3-ylamino)-8-methyl-7-oxopyrido[2,3-d]pyrimidin-6-yl]-2,4-difluorophenyl]-3-fluoropyrrolidine-1-sulfonamide hydrochloride

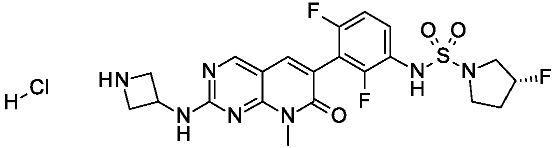

tert-Butyl (R)-3-((6-(2,6-difluoro-3-((3-fluoropyrrolidine)-1-sulfonamido)phenyl)-8-methyl-7-oxo-7,8-dihydropyrido[2,3-d]pyrimidin-2-yl)amino)azetidine-1-carboxylate (intermediate IA6, 26.2 mg, 43 µmol) was dissolved in dioxane (172 µl) and was added to 4M HCl in dioxane (161 µl, 645 µmol) and stirred at rt for 1.5 hours, after which time additional 4M HCl in dioxane (161 µl, 645 µmol) was added and stirring was continued for another 2 hours. The reaction mixture was concentrated *in vacuo* to give 23.4 mg of an off-white solid. LCMS showed that the material had a puritiy of about 60-70%. MS (ESI): 510.3 [(M-HCl+H)⁺].

### Intermediates IA19 - IA25, IA38, IA41

The following intermediates were made in analogy to intermediate IA18 by using the appropriate starting materials and reagents in the reaction:

| **Int.** | **Structure** | **Name** | **Reagent** | **MS (ESI)** |
|---|---|---|---|---|
| IA19 | 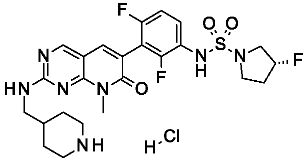 | (3R)-N-[2,4-difluoro-3-[8-methyl-7-oxo-2-(piperidin-4-ylmethylamino)pyrid o[2,3-d]pyrimidin-6-yl]phenyl]-3-fluoropyrrolidine-1-sulfonamide hydrochloride | Intermediate IA8 | 550.5 [(M-HCl-H)⁻] |
| IA20 | 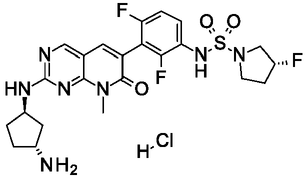 mixture of 2 diastereomers | (3R)-N-[3-[2-[[(1S,3S and 1R,3R)-3-aminocyclopentyl]a mino]-8-methyl-7-oxopyrido[2,3-d]pyrimidin-6-yl]-2,4-difluorophenyl]-3-fluoropyrrolidine-1-sulfonamide hydrochloride | Intermediate IA13 | 536.1 [(M-HCl-H)⁻] |
| IA21 | 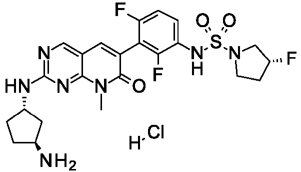 | (3R)-N-[3-[2-[[(1S,3S)-3-aminocyclopentyl]a mino]-8-methyl-7-oxopyrido[2,3-d]pyrimidin-6-yl]-2,4-difluorophenyl]-3-fluoropyrrolidine-1-sulfonamide hydrochloride | Intermediate IA14 | 536.1 [(M-HCl-H)⁻] |
| IA22 | 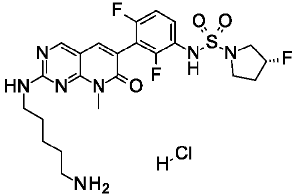 | (3R)-N-[3-[2-(5-aminopentylamino)-8-methyl-7-oxopyrido[2,3-d]pyrimidin-6-yl]-2,4-difluorophenyl]-3-fluoropyrrolidine-1-sulfonamide hydrochloride | Intermediate IA7 | 538.4 [(M-HCl-H)⁻] |
| IA23 | 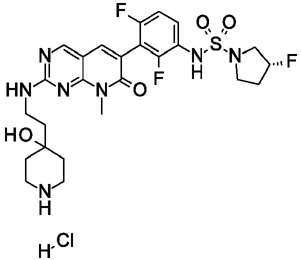 | (3R)-N-[2,4-difluoro-3-[2-[2-(4-hydroxypiperidin-4-yl)ethylamino]-8-methyl-7-oxopyrido[2,3-d]pyrimidin-6-yl]phenyl]-3-fluoropyrrolidine-1-sulfonamide hydrochloride | Intermediate IA15 | 580.5 [(M-HCl-H)⁻] |
| IA24 | 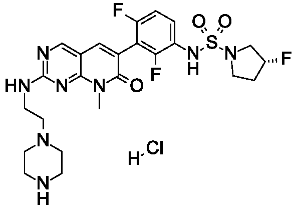 | (3R)-N-[2,4-difluoro-3-[8-methyl-7-oxo-2-(2-piperazin-1-ylethylamino)pyrido [2,3-d]pyrimidin-6-yl]phenyl]-3-fluoropyrrolidine-1-sulfonamide hydrochloride | Example 45 | 565.5 [(M-HCl-H)⁻] |
| IA25 | 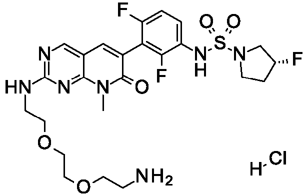 | (3R)-N-[3-[2-[2-[2-(2-aminoethoxy)ethoxy ]ethylamino]-8-methyl-7-oxopyrido[2,3-d]pyrimidin-6-yl]-2,4-difluorophenyl]-3-fluoropyrrolidine-1-sulfonamide hydrochloride | Intermediate IA17 | 584.1 [(M-HCl-H)⁻] |
| IA38 | 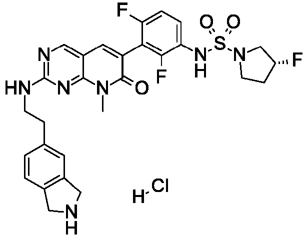 | (3R)-N-[3-[2-[2-(2,3-dihydro-1H-isoindol-5-yl)ethylamino]-8-methyl-7-oxopyrido[2,3-d]pyrimidin-6-yl]-2,4-difluorophenyl]-3-fluoropyrrolidine-1-sulfonamide hydrochloride | Intermediate IA16 | 598.3 [(M-HCl-H)⁻] |
| IA41 | 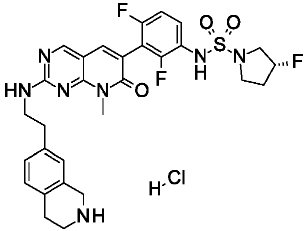 | (3R)-N-[2,4-difluoro-3-[8-methyl-7-oxo-2-[2-(1,2,3,4-tetrahydroisoquinoli n-7-yl)ethylamino]pyrid o[2,3-d]pyrimidin-6-yl]phenyl]-3-fluoropyrrolidine-1-sulfonamide hydrochloride | Intermediate IA40 | 614.3 [MH⁺] |

### Intermediate IA26

### tert-Butyl 4-[4-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]butanoylamino]piperidine-1-carboxylate

### Step 1: 4-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]butanoic acid

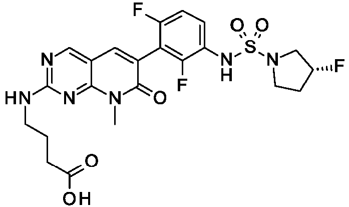

tert-Butyl (R)-4-((6-(2,6-difluoro-3-((3-fluoropyrrolidine)-1-sulfonamido)phenyl)-8-methyl-7-oxo-7,8-dihydropyrido[2,3-d]pyrimidin-2-yl)amino)butanoate (Example 3, 12.3 mg, 20.6 µmol, Eq: 1) was dissolved in DCM (665 µL) and trifluoroacetic acid (47.6 µl, 618 µmol) was added and the reaction mixture was stirred at rt overnight. The reaction was incomplete after that time as judged by LC-MS. The reaction mixture was concentrated in vacuo and then more DCM (500 µL) and trifluoroacetic acid (47.6 µl, 618 µmol) were added. After 7 hours the reaction was still not complete, and so more trifluoroacetic acid (47.6 µl, 618 µmol) was added and stirring was continued overnight. LCMS showed that the reaction was now complete. The mixture was concentrated *in vacuo* to provide the title compound as an orange solid (14.6 mg, 98% yield based on amount and purity) that was used without further purification. MS (ESI): 541.2 [MH⁺].

### Step 2: tert-butyl 4-[4-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]butanoylamino]piperidine-1-carboxylate

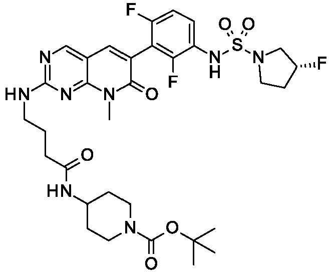

(R)-4-((6-(2,6-difluoro-3-((3-fluoropyrrolidine)-1-sulfonamido)phenyl)-8-methyl-7-oxo-7,8-dihydropyrido[2,3-d]pyrimidin-2-yl)amino)butanoic acid from the previous reaction step (14.6 mg, 20.3 µmol) and HATU (11.6 mg, 30.4 µmol) were dissolved in DMF (101 µl) and stirred for 20 mins. DIPEA (10.6 µl, 60.8 µmol) and tert-butyl 4-aminopiperidine-1-carboxylate (4.46 mg, 22.3 µmol) were added and the reaction was stirred at rt for 10 mins. LC-MS showed the reaction to be complete. The reaction mixture was diluted with ethyl acetate (15 ml) and water (10 ml) and sodium bicarbonate solution (5 ml) and the layers were separated. The aqueous layer was extracted with ethyl acetate (2 x 10 ml). The combined organic layers were washed with water (10 ml) and brine (10 ml), dried over anhydrous sodium sulfate, filtered and concentrated *in vacuo* to give 19.6 mg of a crude product. The crude material was purified by flash chromatography by dissolving it in minimal amount of DCM and applying it to a 4 g silica gel column followed by elution with a gradient of 0-5% MeOH in DCM. The product fractions were combined and concentrated to give 9.9 mg (68%) of the title compound as a waxy solid. MS (ESI): 723.3 [MH⁺].

### Intermediate IA27

### (3R)-N-[3-[2-[2-(3-Cyanophenyl)ethylamino]-8-methyl-7-oxopyrido[2,3-d]pyrimidin-6-yl]-2,4-difluorophenyl]-3-fluoropyrrolidine-1-sulfonamide

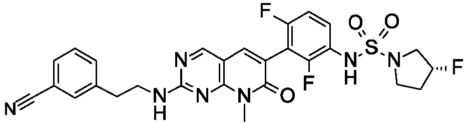

(3R)-N-(2,4-difluoro-3-(8-methyl-2-(methylsulfonyl)-7-oxo-7,8-dihydropyrido[2,3-d]pyrimidin-6-yl)phenyl)-3-fluoropyrrolidine-1-sulfonamide (Intermediate IA1, 50 mg, 96.6 µmol) was dissolved in DMF (623 µl). To the mixture were added 3-(2-aminoethyl)benzonitrile hydrochloride (88.2 mg, 483 µmol) and NEt₃ (67.3 µl, 483 µmol) added and the reaction was stirred at rt for 90 mins. LC-MS confirmed that the reaction was complete. The mixture was diluted with 20 ml ethyl acetate and 20 ml water and the layers were separated. The aqueous layer was extracted with 2x20 ml ethyl acetate and the combined organic layers were washed with 20 ml brine, dried with anhydrous sodium sulfate, filtered and concentrated to give 105.8 mg of a crude product. This material was adsorbed to silica gel, applied to a 12g column and eluted with a gradient of 0-5% MeOH in DCM. Product fractions were combined and concentrated *in vacuo* to give 46.1 mg (82%) of the title compound as a pale yellow powder. MS (ESI): 584.4 [MH⁺].

The following intermediate was made in analogy to Intermediate IA27 by using the appropriate starting materials and reagents:

| **Int.** | **Structure** | **Name** | **Intermediate / Reagent** | **MS (ESI)** |
|---|---|---|---|---|
| IA28 | 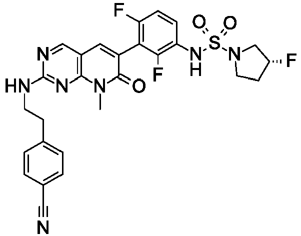 | (3R)-N-[3-[2-[2-(4-cyanophenyl)ethyla mino]-8-methyl-7-oxopyrido[2,3-d]pyrimidin-6-yl]-2,4-difluorophenyl]-3-fluoropyrrolidine-1-sulfonamide | Intermediate IA1 and 4-(2-aminoethyl)be nzonitrile | 584.3 [MH⁺] |

### Intermediate IA29

### tert-Butyl N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]-N-methylcarbamate

### Step 1: tert-Butyl N-methyl-N-[[3-[2-(phenylmethoxycarbonylamino)ethyl]phenyl]methyl]carbamate

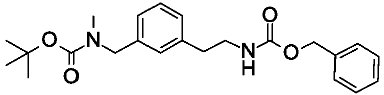

tert-Butyl (3-Bromobenzyl)(methyl)carbamate (316 mg, 1.05 mmol), potassium (2-(((benzyloxy)carbonyl)amino)ethyl)trifluoroborate (303 mg, 1.06 mmol), cesium carbonate (1.03 g, 3.16 mmol), toluene (4.93 ml) and water (1.64 ml) were added to a vial and argon was bubbled through the suspension for 5 min. Then, 1,1'-bis(diphenylphosphino)ferrocene-palladium(II)dichloride dichloromethane complex (43 mg, 52.6 µmol) was added and argon was bubbled through the suspension for 1 min. The vial was sealed and the reaction mixture was stirred at 80 °C for 4 hr. Starting material and product have the same retention time, so only about 50% conversion after 4h could be observed. The reaction mixture was partitioned between DCM (20 ml) and a saturated aqueous ammonium chloride solution (20 ml). The organic layer was separated and the aqueous layer was extracted with DCM (3x20 ml). The combined organic layer was washed with water (30 mL), brine (30 mL), dried over magnesium sulfate, filtered and concentrated *in vacuo.* The resulting residue was purified using flash column chromatography (silica gel, 24 g, gradient of EtOAc in heptane 0-50%) to yield the title compound as a colorless oil. ¹H NMR (CDCl₃, 300 MHz); 7.3-7.4 (m, 5H), 7.1-7.3 (m, 1H), 7.0-7.1 (m, 3H), 5.09 (s, 2H), 4.39 (br s, 2H), 3.46 (q, 2H, J=6.7 Hz), 2.81 (br t, 5H, J=6.7 Hz), 1.47 (s, 9H).

### Step 2: tert-butyl N-[[3-(2-aminoethyl)phenyl]methyl]-N-methylcarbamate

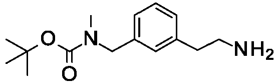

To a solution of tert-butyl (3-(2-(((benzyloxy)carbonyl)amino)ethyl)benzyl)(methyl)carbamate (170 mg, 427 µmol) obtained in the previous reaction step in ethyl acetate (10 ml) under argon was added palladium on carbon (22.7 mg, 21.3 µmol). The atmosphere was exchanged to hydrogen with a balloon and stirring was continued. All of the starting material was consumed after 18h. The reaction mixture was filtered through a pad of celite and the filter cake was washed with EtOAc (5x10 mL). The filtrate was concentrated to give the product as a colorless oil. ¹H NMR (CDCl₃, 300 MHz); 7.2-7.3 (m, 2H), 7.0-7.1 (m, 3H), 4.40 (s, 2H), 2.9-3.0 (m, 2H), 2.82 (br d, 3H, J=6.4 Hz), 2.7-2.8 (m, 2H), 1.48 (s, 9H), 1.2-1.3 (m, 2H).

### Step 3: tert-Butyl N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]-N-methylcarbamate

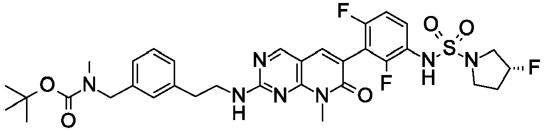

This material was made in analogy to intermediate IA6 by using intermediate IA1 (124 mg, 192 µmol) and tert-butyl N-[[3-(2-aminoethyl)phenyl]methyl]-N-methylcarbamate (110 mg, 416 µmol) as starting materials. The title compound was obtained as a brown solid: 42.4 mg (32%). MS (ESI): 702.7 [MH⁺].

### Intermediate IA30

### tert-Butyl 4-[2-[[6-[2-cyano-3-[[ethyl(methyl)sulfamoyl]amino]-phenyl]-8-methyl-7-oxopyrido[2,3-d]pyrimidin-2-yl]amino]ethyl]piperidine-1-carboxylate

### Step1: Ethyl 2-(3-bromo-2-cyanophenyl)acetate

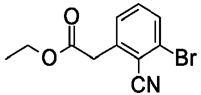

To a suspension of sodium hydride (660 mg, 16.5 mmol) (pre-washed with heptane to remove mineral oil) in THF (7.5 ml) was added diethyl malonate (2.5 ml, 16.5 mmol) in THF (7.5 ml) dropwise at 0 °C. 2-bromo-6-fluorobenzonitrile (3.0 g, 15 mmol) was then added and the reaction was warmed to rt with stirring. After 1.5 h there was no indication of substantial conversion. The reaction was then heated at reflux and after 1.5 h approx. a 2:1 ratio of SM and diester product was observed and the reaction was heated to reflux for another 20 hours. At that time, some solid material was present in the reaction mixture. The reaction was cooled to rt and most of the THF was removed *in vacuo.* The residue was diluted with 1 M aq. HCl (150 mL) and was extracted with TBME (3 x 150 mL). The combined organic extracts were washed with water (300 mL) and brine (300 mL), dried (MgSO₄), filtered and concentrated *in vacuo* to provide 5.12 g of a yellow oil that contained the diester intermediate.

To a solution of this crude intermediate in DMSO (15 mL) and water (351 µl) was added lithium chloride (668 mg, 15.7 mmol), and the reaction was stirred at 120 °C for 1.5 h and was then cooled to rt. At that time LC-MS suggested that only small amount of diester was remaining. The solution was diluted with water (150 mL) and was extracted with TBME (3 x 150 mL). The combined organic layers were washed with water (200 mL) and brine (200 mL), dried (MgSO₄), filtered and concentrated *in vacuo* to afford 4.3 g of a yellow oil. This material was purified by silica gel chromatography (120 g column) by elution with a gradient of 10-20% EtOAc/heptanes and the fractions containing the desired material were combined and evaporated to afford 1.71 g (32%) of the title compound as colourless crystals with 75% purtiy (UV). LC-MS suggested that diester is present (12%). MS (ESI): 268.0 [MH⁺].

### Step 2: 2-Bromo-6-(8-methyl-2-methylsulfanyl-7-oxopyrido[2,3-d]pyrimidin-6-yl)benzonitrile

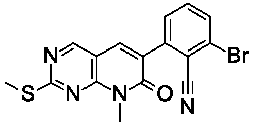

This material was made in analogy to the method described by Ren et al., Bioorg. Med. Chem. Lett. 22 (2012), 3387. Ethyl 2-(3-bromo-2-cyanophenyl)acetate (obtained in the previous step, 1 g, 2.8 mmol) and 4-(methylamino)-2-(methylthio)pyrimidine-5-carbaldehyde (513 mg, 2.8 mmol) were dissolved in DMA (14 ml). Potassium fluoride (40% on alumina) (2.84 g, 19.6 mmol) was added, and the reaction mixture was stirred at rt for one hour. After this time LC-MS suggested mainly product and only traces of SM. After 2 h, the reaction was filtered and the solids were washed with DMA. Then, the filtrate was concentrated *in vacuo.* The dark red residue was triturated with boiling EtOH, filtered and washed with further EtOH and heptanes. This provided 537 mg (50%) of the title compound as a pale pink solid with 100% purity. MS (ESI): 387.0 [MH⁺].

### Step 3: 2-Bromo-6-(8-methyl-2-methylsulfonyl-7-oxopyrido[2,3-d]pyrimidin-6-yl)benzonitrile

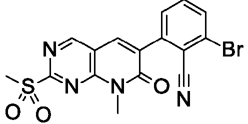

This material was made in analogy to Intermediate IA1, step 2, by treatment of 2-bromo-6-(8-methyl-2-methylsulfanyl-7-oxopyrido[2,3-d]pyrimidin-6-yl)benzonitrile (535 mg, 1.38 mmol), obtained in the previous step, with a total of 894 mg (3.94 mmol) m-CPBA in DMF (10 ml). The title compound was obtained as a light yellow solid (521 mg, 68%). MS (ESI): 419.1 [MH⁺].

### Step 4: tert-Butyl 4-[2-[[6-(3-bromo-2-cyanophenyl)-8-methyl-7-oxopyrido[2,3-d]pyrimidin-2-yl]amino]ethyl]piperidine-1-carboxylate

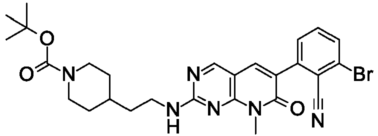

2-Bromo-6-(8-methyl-2-(methylsulfonyl)-7-oxo-7,8-dihydropyrido[2,3-d]pyrimidin-6-yl)benzonitrile (515 mg, 921 µmol), obtained in the previous step, was suspended in dioxane (4.61 ml). DIPEA (322 µl, 1.84 mmol) and tert-butyl 4-(2-aminoethyl)piperidine-1-carboxylate (421 mg, 1.84 mmol) were added, and the reaction mixture was stirred at rt. After 30 min LC-MS suggests complete consumption of the starting material. The reaction mixture was diluted with EtOAc (30 mL) and washed with 10% aq. citric acid (2 x 30 mL), water (30 mL), sat. aq. NaHCO₃ (30 mL) and brine (30 mL). The organic layer was concentrated *in vacuo* to a volume of around 10 mL, where a precipitate was formed. This solid was collected by filtration and was washed with water, toluene and heptane. The material was dried in vacuo to afford 504 mg (96%) of the title compound as a pale yellow solid. MS (ESI): 511.1 [(M+H-^{tert}Bu)⁺].

### Step 5: tert-Butyl 4-[2-[[6-[2-cyano-3-[[ethyl(methyl)sulfamoyl]amino]-phenyl]-8-methyl-7-oxopyrido[2,3-d]pyrimidin-2-yl]amino]ethyl]piperidine-1-carboxylate

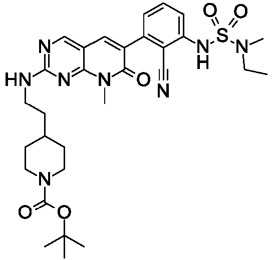

tert-Butyl 4-(2-((6-(3-bromo-2-cyanophenyl)-8-methyl-7-oxo-7,8-dihydropyrido[2,3-d]pyrimidin-2-yl)amino)ethyl)piperidine-1-carboxylate (100 mg, 176 µmol), obtained in the previous step, [ethyl(methyl)amino]sulfamide (24.4 mg, 176 µmol) and cesium carbonate (74.6 mg, 229 µmol) were added to a dry 0.5-2 mL microwave vial. Dry dioxane (881 µl) was added, and the reaction was sparged with argon. Pd₂(dba)₃ (8.07 mg, 8.81 µmol) and 2- dicyclohexylphosphino-2'-(N,N-dimethylamino)biphenyl (10.4 mg, 26.4 µmol) were added, and the reaction was again purged with argon. The vial was sealed, and the reaction was heated at 80 °C overnight. After 16 h, LC-MS suggested the presence of starting material, dehalogenated starting material and the deisired product. Therefore, the reaction was then heated to 100 °C. After 6 h starting material was still visible. More [ethyl(methyl)amino]sulfamide (24.4 mg, 176 µmol, Eq: 1), Pd₂(dba)₃ (8.07 mg, 8.81 µmol, Eq: 0.05) and 2-dicyclohexylphosphino-2'-(N,N-dimethylamino)biphenyl (10.4 mg, 26.4 µmol, Eq: 0.15) were added, and the reaction was heated to 100 °C. After another 16 h only product and dehalogenated starting material were visible. The reaction mixture was cooled to rt and filtered through cotton wool, eluting with EtOAc containing a few drops of AcOH. The filtrate was concentrated onto Isolute, and purified by silica gel chromatography (20 g, gradient of 0-3% MeOH/DCM). Fractions containing the desired material were combined and evaporated to provide the title compound as a brown solid (36 mg, 92% purity (UV), 30% yield). MS (ESI): [(M+H-^{tert}Bu)⁺].

### Intermediate IA31

### tert-Butyl N-[2-[2-[2-[2-(cyclopropylmethylamino)-6-[2,6-difluoro-3-[[rac-(3R)-3-fluoropyrrolidin-1-yl]sulfonylamino]phenyl]-7-oxopyrido[2,3-d]pyrimidin-8-yl]ethoxy]ethoxy]ethyl]carbamate

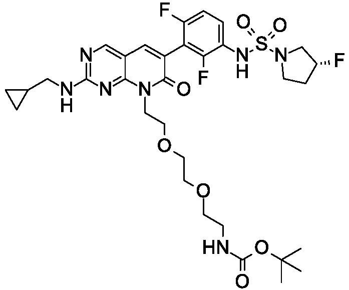

### Step 1: Ethyl 4-[2-[2-[2-[(2-methylpropan-2-yl)oxycarbonylamino]ethoxy]ethoxy]ethylamino]-2-methylsulfanylpyrimidine-5-carboxylate

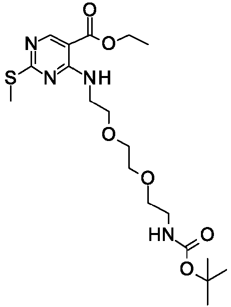

Ethyl 4-chloro-2-(methylthio)pyrimidine-5-carboxylate (650 mg, 2.79 mmol) was dissolved in THF (10.4 ml). Then, triethylamine (1.44 ml, 10.3 mmol) and tert-butyl (2-(2-(2-aminoethoxy)ethoxy)ethyl)carbamate (995 µl, 4.19 mmol) were added to the solution at 0 C, and the reaction was stirred for 50 mins. LC-MS showed complete consumption of the starting material. The reaction mixture was diluted with water (30 ml) and ethyl acetate (30 ml) and the layers were separated. The aqueous layer was extracted with 2x 30 ml ethyl acetate and the organic layers washed with 30 ml brine. The organic extracts were dried with anhydrous Na₂SO₄, filtered and concentrated to yield 1.33 g of a crude yellow oil. This material was purified by flash chromatography by dissolving it a minimal amount of DCM and applying it to an 80 g column, followed by elution with a gradient of 0-50% ethyl acetate in heptane. Fractions containing the product were combined and evaporated *in vacuo* to give 800.6 mg (65 %) of the title compound as a clear oil. MS (ESI): 445.3 [MH⁺] .

### Step 2: tert-Butyl N-[2-[2-[2-[[5-(hydroxymethyl)-2-methylsulfanylpyrimidin-4-yl]amino]ethoxy]ethoxy]ethyl]carbamate

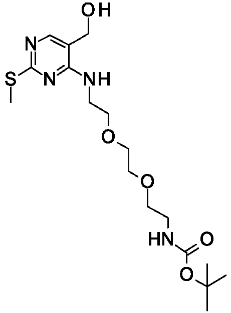

Ethyl 4-((2,2-dimethyl-4-oxo-3,8,11-trioxa-5-azatridecan-13-yl)amino)-2-(methylthio)pyrimidine-5-carboxylate (798.4 mg, 1.8 mmol) was dissolved in THF (5 ml) and cooled to 0 C. LiAlH₄ 1 M solution in THF (1.98 ml, 1.98 mmol) added slowly over 45 mins and the reaction mixture was allowed to stir at 0 C for another 2.5 hours. Then, the mixture was slowly quenched with 80 µL water, followed by addition of 80 µL of a 15% aq NaOH solution. The suspension was filtered and washed with THF and the filtrate was concentrated to yield 632.6 mg of a cloudy yellow oil. This material was purified by flash chromatography by applying it to a 40 g column followed by elution with a gradient of 0-5% MeOH in DCM. Product fractions were combined and evaporated to give 468.5 mg (65%) of the title compound as a pale yellow oil. MS (ESI): 403.3 [MH⁺].

### Step 3: tert-Butyl N-[2-[2-[2-[(5-formyl-2-methylsulfanylpyrimidin-4-yl)amino]ethoxy]ethoxy]ethyl]carbamate

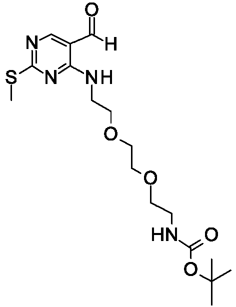

tert-Butyl (2-(2-(2-((5-(hydroxymethyl)-2-(methylthio)pyrimidin-4-yl)amino)ethoxy)ethoxy)ethyl)carbamate (468.5 mg, 1.16 mmol) was dissolved in DCM (14 ml). Manganese dioxide (1.01 g, 11.6 mmol) was added and the suspension was stirred at rt for 4.5 hours. LC-MS showed complete consumption of the starting material. The mixture was filtered through a celite pad and the filter cake was rinsed with DCM. The filtrate was concentrated in vacuo to yield 465.4 mg of a cloudy oil. This crude material was dissolved in a minimal amount of DCM wand was applied to a 25 g column, followed by elution with a gradient of 0-50% ethyl acetate in heptane. Fractions containing pure product fractions were combined and evaporated to provide the title compound (426.2 mg, 91%) as a pale yellow cloudy oil. MS (ESI): 401.2 [MH⁺].

### Step 4: tert-Butyl N-[2-[2-[2-[6-(3-amino-2,6-difluorophenyl)-2-methylsulfanyl-7-oxopyrido[2,3-d]pyrimidin-8-yl]ethoxy]ethoxy]ethyl]carbamate

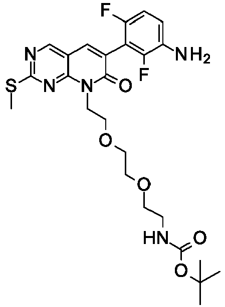

tert-Butyl (2-(2-(2-((5-formyl-2-(methylthio)pyrimidin-4-yl)amino)ethoxy)ethoxy)ethyl)carbamate (424.2 mg, 1.06 mmol) and methyl 2-(3-amino-2,6-difluorophenyl)acetate (320 mg, 1.59 mmol) were dissolved in DMF (13 ml) and K₂CO₃ (732 mg, 5.3 mmol) was added and the mixture was stirred at 80 °C for 5 hours. The reaction was progressing slowly, but only little ester remained so more methyl 2-(3-amino-2,6-difluorophenyl)acetate (213 mg, 1.06 mmol) was added and the temperature was increased to 90 °C. Stirring was continued overnight but the reaction was still not complete, so additional methyl 2-(3-amino-2,6-difluorophenyl)acetate (107 mg, 530 µmol) was added. After another 2 hours, another amount of methyl 2-(3-amino-2,6-difluorophenyl)acetate (107 mg, 530 µmol) was added and the temperature was increased to 100 °C and the reaction was stirred overnight again. The reaction mixture was then concentrated ini vacuo to remove most of the DMF and was then diluted with 40 ml ethyl acetate and 40 ml 2M Na₂CO₃ and the layers were separated. The aqueous layer was extracted with 2 x 30 ml ethyl acetate and the combined organic layers washed with 30 ml brine. The organic extract was dried with anhydrous Na₂SO₄, filtered and concentrated in vacuo and the remainder of the DMF was removed by co-evaporation with 2 x 50 ml toluene. This afforded 1.16 g of a brown oil. This material was dissolved in a minimal amount of DCM and was applied to a 40 g silica gel column, followed by elution with a gradient of 0 - 70 % ethyl acetate in heptane. Product fractions were combined and concentrated in vacuo to give 335 mg (57%) of the title compound as a light brown foam. MS (ESI): 552.3 [MH⁺].

### Step 5: tert-Butyl N-[2-[2-[2-[6-[2,6-difluoro-3-[[(3R)-3-fluoropyrrolidin-1-yl]sulfonylamino]phenyl]-2-methylsulfanyl-7-oxopyrido[2,3-d]pyrimidin-8-yl]ethoxy]ethoxy]ethyl]carbamate

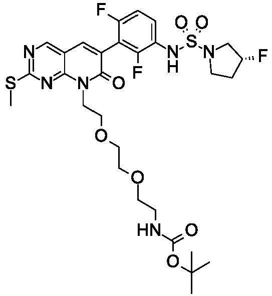

DCM (2.9 ml) was added to tert-butyl (2-(2-(2-(6-(3-amino-2,6-difluorophenyl)-2-(methylthio)-7-oxopyrido[2,3-d]pyrimidin-8(7H)-yl)ethoxy)ethoxy)ethyl)carbamate (332 mg, 602 µmol). At rt, pyridine (2.23 ml, 27.7 mmol), DMAP (3.75 mg, 30.1 µmol) and (R)-3-fluoropyrrolidine-1-sulfonyl chloride (226 mg, 1.2 mmol) were added. The reaction mixture was then refluxed overnight at 60 °C. The reaction mixture was cooled and diluted with 30 ml methyl-THF and a small amount of ice. Saturated aq. NaHCO₃ solution was added (30 ml) and the layers were separated. The aqueous layer was extracted with 2 x 30 ml methyl-THF. The combined organic layers were washed with 30 ml brine, dried with anhydrous Na₂SO₄, filtered and concentrated in vacuo to afford 578.2 mg of a brown oil. This material was purified by flash chromatography by dissolving the residue in a minimal amount of DCM and applying it to a 40 g column, followed by elution with a gradient of 0-70% ethyl acetate in heptane. Product fractions were combined and concentrated *in vacuo* to afford 210.3 mg (50%) of the title compound as a light brown oil. MS (ESI): 701.4 [(M-H)⁻].

### Step 6: tert-Butyl N-[2-[2-[2-[6-[2,6-difluoro-3-[[(3R)-3-fluoropyrrolidin-1-yl]sulfonylamino]phenyl]-2-methylsulfonyl-7-oxopyrido[2,3-d]pyrimidin-8-yl]ethoxy]ethoxy]ethyl]carbamate

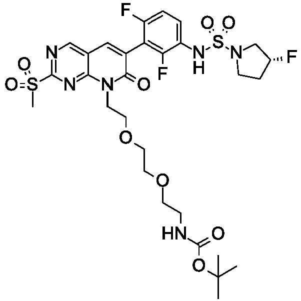

tert-Butyl (R)-(2-(2-(2-(6-(2,6-difluoro-3-((3-fluoropyrrolidine)-1-sulfonamido)phenyl)-2-(methylthio)-7-oxopyrido[2,3-d]pyrimidin-8(7H)-yl)ethoxy)ethoxy)ethyl)carbamate (210.3 mg, 299 µmol) was dissolved in DCM (3.2 ml) and m-CPBA (103 mg, 598 µmol) was added. The mixture was then stirred at rt for 1.5 hours. About 50% of mono-oxidised product was still present, so more m-CPBA (51.6 mg, 299 µmol) was added and the reaction mixture stirred for another hour. Now, LC-MS showed completion of the reaction. The reaction mixture was diluted with 20 ml DCM and 20 ml sat NaHCO₃ and the layers were separated. The aqueous layer was extracted with 2 x 20 ml DCM. The organic extracts were washed with 20 ml water, dried with anhydrous Na₂SO₄, filtered and concentrated *in vacuo* to afford 204.6 mg of a crude yellow oil. This material was dissolved in a minimal amount of DCM and was applied to a 25 g column. The column was eluted with a gradient of 0-5% MeOH in DCM. Product fractions were combined and concentrated *in vacuo* to give 125.7 mg (57%) of the title compound as a pale yellow foam. MS (ESI): 733.4 [(M-H)⁻].

### Step 7: tert-Butyl N-[2-[2-[2-[2-(cyclopropylmethylamino)-6-[2,6-difluoro-3-[[(3R)-3-fluoropyrrolidin-1-yl]sulfonylamino]phenyl]-7-oxopyrido[2,3-d]pyrimidin-8-yl]ethoxy]ethoxy]ethyl]carbamate

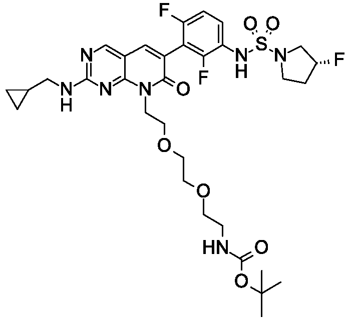

tert-Butyl (R)-(2-(2-(2-(6-(2,6-difluoro-3-((3-fluoropyrrolidine)-1-sulfonamido)phenyl)-2-(methylsulfonyl)-7-oxopyrido[2,3-d]pyrimidin-8(7H)-yl)ethoxy)ethoxy)ethyl)carbamate (60 mg, 81.7 µmol) was dissolved in dioxane (700 µl) and aminomethylcyclopropane (35.4 µl, 408 µmol) was added. The reaction was then stirred for 20 min at rt. The mixture was concentrated *in vacuo* to afford 102.5 mg of a crude yellow oil. This material was dissolved in a minimal amount of DCM and was applied to a 12 g column, followed by elution with a gradient of 0-70% ethyl acetate in heptane. Product fractions were combined and concentrated *in vacuo* to give 58 mg (98%) of the title compound as a yellow oil. MS (ESI): 724.5 [(M-H)⁻].

The following intermediates were made in analogy to example 38 by using the appropriate starting materials and the corresponding reagents:

| **Int.** | **Structure** | **Name** | **Starting material** | **MS (ESI)** |
|---|---|---|---|---|
| IA32 | 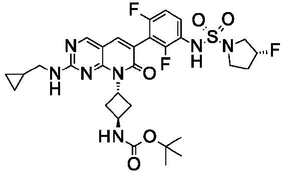 | trans-tert-butyl N-[3-[2-(cyclopropylmethylamin o)-6-[2,6-difluoro-3-[[(3R)-3-fluoropyrrolidin-1-yl]sulfonylamino]phenyl] -7-oxopyrido[2,3-d]pyrimidin-8-yl]cyclobutyl]carbamate | In step 1: trans tert-butyl N-(3-aminocyclo butyl)carba mate | 664.5 [MH⁺] |

### Intermediate IA33

### (3R)-N-[3-[8-(trans-3-aminocyclobutyl)-2-(cyclopropylmethylamino)-7-oxopyrido[2,3-d]pyrimidin-6-yl]-2,4-difluorophenyl]-3-fluoropyrrolidine-1-sulfonamide hydrochloride

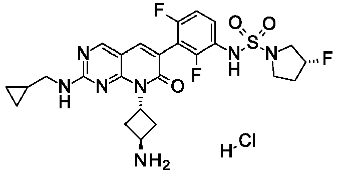

The title compound was made in analogy to Intermediate IA18 from Intermediate IA32 by treatment with HCl in dioxane as an off-white solid: MS (ESI): 562.5 [(M-HCl-H)⁻].

### Intermediate IA34

### tert-Butyl 5-(2-aminoethyl)-1,3-dihydroisoindole-2-carboxylate

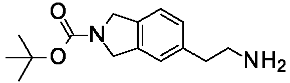

### Step1: tert-Butyl 5-[2-(phenylmethoxycarbonylamino)ethyl]-1,3-dihydroisoindole-2-carboxylate

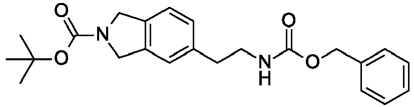

A suspension of tert-butyl 5-bromoisoindoline-2-carboxylate (400 mg, 1.34 mmol), potassium (2-(((benzyloxy)carbonyl)amino)ethyl)trifluoroborate (402 mg, 1.41 mmol) and cesium carbonate (1.31 g, 4.02 mmol)in toluene (6.29 ml) and water (2.1 ml) was sparged with argon for 10 min. Then, methanesulfonato(2-dicyclohexylphosphino-2',6'-di-i-propoxy-1,1'-biphenyl)(2-amino-1,1'-biphenyl-2-yl)palladium(II) (56.1 mg, 67.1 µmol) was added and the reaction mixture was sparged with argon for another 3 min. The vial was sealed and heated to 80 °C over night. LC-MS shows complete conversion after 16h. The reaction mixture was filtered through a pad of celite and washed with EtOAc (80 mL). The filtrate was concentrated, applied onto isolute and purified using flash column chromatography (silica gel, 40 g, gradient of EtOAc in heptane 0-50%) to yield the product as a yellow oil (332 mg, 63%). MS (ESI): 297.2 [(M+H-BOC)⁺].

### Step 2: tert-Butyl 5-(2-aminoethyl)-1,3-dihydroisoindole-2-carboxylate

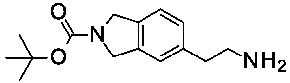

A solution of tert-butyl 5-(2-(((benzyloxy)carbonyl)amino)ethyl)isoindoline-2-carboxylate (334.2 mg, 843 µmol) in EtOAc (28.1 ml) was placed under argon. Next, palladium on activated carbon (10% loading, 44.9 mg) was added, the flask was evacuated briefly and backfilled with argon. Then the atmosphere was exchanged for hydrogen and the reaction mixture was stirred at rt for 16h. The reaction mixture was filtered through a pad of celite and was washed with EtOAc (5x10 mL). The filtrate was concentrated to yield the product as a colorless oil (221 mg, 96%). MS (ESI): 263.4 [MH⁺].

The following intermediates were made in analogy to intermediate IA34, steps 1 and 2 by using the appropriate starting materials and the corresponding reagents:

| **Int.** | **Structure** | **Name** | **Starting material** | **MS (ESI)** |
|---|---|---|---|---|
| IA39 | 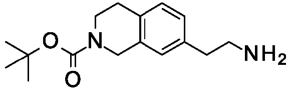 | tert-butyl 7-(2-aminoethyl)-3,4-dihydro-1H-isoquinoline-2-carboxylate | In step 1: tert-butyl 7-bromo-3,4-dihydroisoqui noline-2(1H)-carboxylate | 277.2 [MH⁺] |

### Intermediate IA35

### Methyl 2-[3-(2-aminoethyl)phenyl]acetate

### Step 1: Methyl 2-[3-[2-(phenylmethoxycarbonylamino)ethyl]phenyl]acetate

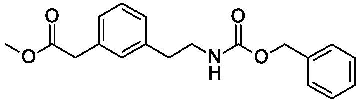

To a vial was added methyl 2-(3-bromophenyl)acetate (300 mg, 1.31 mmol), potassium (2-(((benzyloxy)carbonyl)amino)ethyl)trifluoroborate (377 mg, 1.32 mmol) and cesium carbonate (1.28 g, 3.93 mmol). After addition of toluene (6.14 ml) and water (2.05 ml), argon was bubbled through the suspension for 5 min. Then, 1,1'-bis(diphenylphosphino)ferrocene-palladium(II)dichloride dichloromethane complex 53.5 mg, 65.5 µmol) was added and argon was bubbled through the suspension for 1 min. The vial was sealed and the reaction was stirred at 80 °C for 17h. Then, the reaction mixture was partitioned between an ammonium chloride solution (15 ml) and DCM (15 ml). The organic layer was separated and the aqueous layer was extracted with more DCM (3x15 mL). The combined organic layers were dried over magnesium sulfate, filtered and concentrated *in vacuo.* The residue was dissolved in a minimal amount of DCM and purified using flash column chromatography (silica gel, 20 g, gradient of EtOAc in heptane 0-50%) to provide the title compound as a colorless oil (275 mg, 64%). MS (ESI): 328.2 [MH⁺].

### Step 2: Methyl 2-[3-(2-aminoethyl)phenyl]acetate

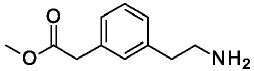

This material was made in analogy to intermediate IA34, step 2, from methyl 2-[3-[2-(phenylmethoxycarbonylamino)ethyl]phenyl]acetate (265 mg, 0.809 mmol) by hydrogenation with Pd on activated charcoal (10% loading) The title compound was obtained as a colorless oil (159 mg, 97%). MS (ESI): 194.1 [MH⁺].

The following intermediates were made in analogy to intermediate IA34, steps 1 and 2, by using the appropriate starting materials and the corresponding reagents:

| **Int.** | **Structure** | **Name** | **Starting material** | **MS (ESI)** |
|---|---|---|---|---|
| IA36 | 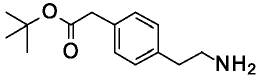 | tert-butyl 2-[4-(2-aminoethyl)phenyl]ac etate | In step 1: tert-butyl 2-(4-bromophenyl)a cetate | 236.2 [MH⁺] |
| IA37 | 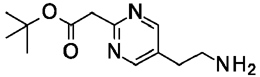 | tert-butyl 2-[5-(2-aminoethyl)pyrimidin-2-yl]acetate | In step 1: tert-butyl 2-(5-bromopyrimidi n-2-yl)acetate | 238.1 [MH⁺] |

### Example 1

### tert-Butyl 4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]piperidine-1-carboxylate

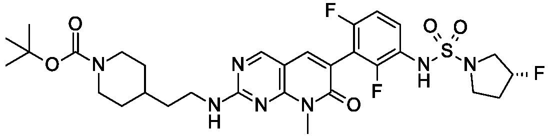

(R)-N-(2,4-difluoro-3-(8-methyl-2-(methylsulfonyl)-7-oxo-7,8-dihydropyrido[2,3-d]pyrimidin-6-yl)phenyl)-3-fluoropyrrolidine-1-sulfonamide (Intermediate IA1, 450 mg, 870 µmol) was dissolved in dioxane (5.8 ml) and tert-butyl 4-(2-aminoethyl)piperidine-1-carboxylate (993 mg, 4.35 mmol) was added. The mixture was stirred for 30 mins at rt and LC-MS showed that the reaction had completed. The mixture was concentrated *in vacuo,* and the residue was dissolved in a minimal amount of DCM. The solution was applied to a 40 g silica gel column and the column and eluted with a gradient of 0-5% MeOH in DCM. Fraction s containing pure product were combined and evaporated to give 508 mg (88%) of the desired material as a pale yellow solid. MS (ESI⁻): 664.3 [(M-H)⁻].

### Examples 2 - 13, 45 - 48

The following examples were made in analogy to example 1, by using the appropriate intermediates and reagents:

| **Ex.** | **Structure** | **Name** | **Intermediate / Reagents** | **MS (ESI)** |
|---|---|---|---|---|
| 2 | 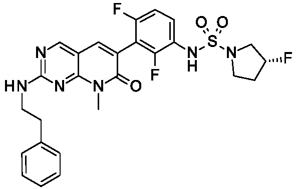 | (3R)-N-[2,4-difluoro-3-[8-methyl-7-oxo-2-(2-phenylethylamino)pyrido [2,3-d]pyrimidin-6-yl]phenyl]-3-fluoropyrrolidine-1-sulfonamide | Intermediate IA1 and | 559.2 [MH⁺] |
| | | | 2-phenylethan-1-amine | |
| 3 | 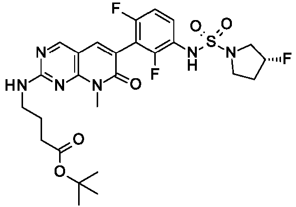 | tert-butyl 4-[[6-[2,6-difluoro-3-[[(3R)-3-fluoropyrrolidin-1-yl]sulfonylamino]phenyl ]-8-methyl-7-oxopyrido[2,3-d]pyrimidin-2-yl]amino]butanoate | Intermediate IA1 and | 597.3 [MH⁺] |
| | | | tert-butyl 4-aminobutanoate hydrochloride | |
| | | | DMF instead of dioxane used as solvent | |
| 4 | 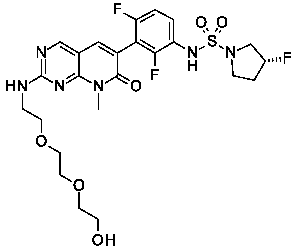 | (3R)-N-[2,4-difluoro-3-[2-[2-[2-(2-hydroxyethoxy)ethoxy] ethylamino]-8-methyl-7-oxopyrido[2,3-d]pyrimidin-6-yl]phenyl]-3-fluoropyrrolidine-1-sulfonamide | Intermediate IA1 and | 587.2 [MH⁺] |
| | | | 2-(2-(2-aminoethoxy) ethoxy)ethan-1-ol | |
| 5 | 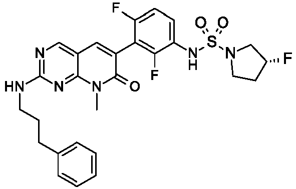 | (3R)-N-[2,4-difluoro-3-[8-methyl-7-oxo-2-(3-phenylpropylamino)pyrido [2,3-d]pyrimidin-6-yl]phenyl]-3-fluoropyrrolidine-1-sulfonamide | Intermediate IA1 and | 573.2 [MH⁺] |
| | | | 3-phenylpropan -1-amine | |
| 6 | 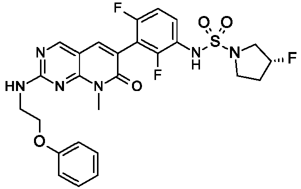 | (3R)-N-[2,4-difluoro-3-[8-methyl-7-oxo-2-(2-phenoxyethylamino) pyrido[2,3-d]pyrimidin-6-yl]phenyl]-3-fluoropyrrolidine-1-sulfonamide | Intermediate IA1 and | 575.2 [MH⁺] |
| | | | 2-phenoxyethan -1-amine | |
| 7 | 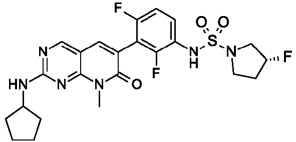 | (3R)-N-[3-[2-(cyclopentylamino)-8-methyl-7-oxopyrido[2,3-d]pyrimidin-6-yl]-2,4-difluorophenyl]-3-fluoropyrrolidine-1-sulfonamide | Intermediate IA1 and | 523.2 [MH⁺] |
| | | | cyclopentanamine | |
| 8 | 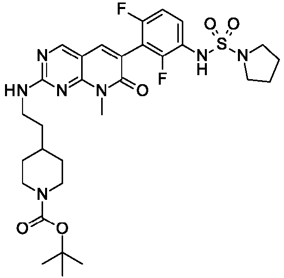 | tert-butyl 4-[2-[[6-[2,6-difluoro-3-(pyrrolidin-1-ylsulfonylamino)phenyl ]-8-methyl-7-oxopyrido[2,3-d]pyrimidin-2-yl]amino]ethyl]piperidine -1-carboxylate | Intermediate IA2 and | 646.8 [(M-H)⁻] |
| | | | tert-butyl 4-(2-aminoethyl) piperidine-1-carboxylate | |
| 9 | 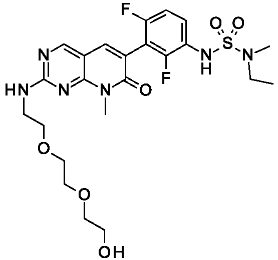 | 6-[3-[[ethyl(methyl)sulfamo yl]amino]-2,6-difluorophenyl]-2-[2-[2-(2-hydroxyethoxy)ethoxy] ethylamino]-8-methyl-7-oxopyrido[2,3-d]pyrimidine | Intermediate IA3 and | 557.3 [MH⁺] |
| | | | 2-(2-(2-aminoethoxy) ethoxy)ethan-1-ol | |
| 10 | 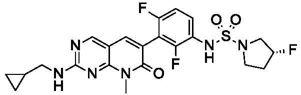 | (3R)-N-[3-[2-(cyclopropylmethylamino )-8-methyl-7-oxopyrido[2,3-d]pyrimidin-6-yl]-2,4-difluorophenyl]-3-fluoropyrrolidine-1-sulfonamide | Intermediate IA1 and cyclopropylm ethanamine | 509.3 [MH⁺] |
| 11 | 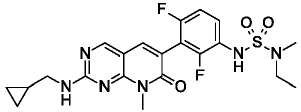 | 2-(cyclopropylmethylamino )-6-[3-[[ethyl(methyl)sulfamoyl ]amino]-2,6-difluorophenyl]-8-methyl-7-oxopyrido[2,3-d]pyrimidine | Intermediate IA3 and cyclopropylmethanamine | 479.3 [MH⁺] |
| 12 | 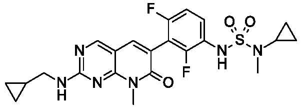 | 2-(cyclopropylmethylamino )-6-[3-[[cyclopropyl(methyl) sulfamoyl]amino]-2,6-difluorophenyl]-8-methyl-7-oxopyrido[2,3-d]pyrimidine | Intermediate IA4 and cyclopropylmethanamine | 491.3 [MH⁺] |
| 13 | 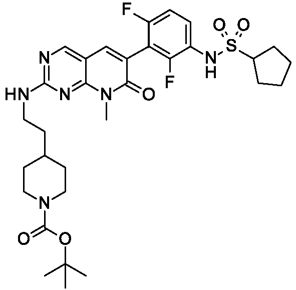 | tert-butyl 4-[2-[[6-[3-(cyclopentylsulfonylamino )-2,6-difluorophenyl]-8-methyl-7-oxopyrido[2,3-d]pyrimidin-2-yl]amino]ethyl]piperidine -1-carboxylate | Intermediate IA5 and | 645.6 [M-H]⁻ |
| | | | tert-butyl 4-(2-aminoethyl) piperidine-1-carboxylate | |
| 45 | 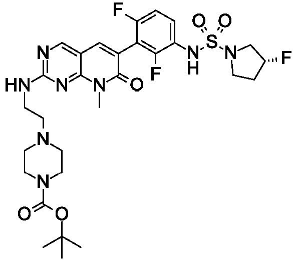 | tert-butyl 4-[2-[[6-[2,6-difluoro-3-[[(3R)-3-fluoropyrrolidin-1-yl]sulfonylamino]phenyl ]-8-methyl-7-oxopyrido[2,3-d]pyrimidin-2-yl]amino]ethyl]piperazine -1-carboxylate | Intermediate IA1 and | 667.6 [MH⁺] |
| | | | tert-butyl 4-(2-aminoethyl)p iperazine-1-carboxylate | |
| 46 | 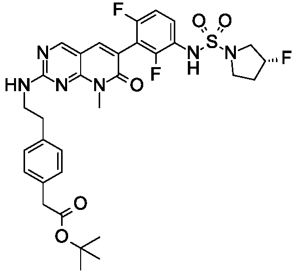 | tert-butyl 2-[4-[2-[[6-[2,6-difluoro-3-[[(3R)-3-fluoropyrrolidin-1-yl]sulfonylamino]phenyl ]-8-methyl-7-oxopyrido[2,3-d]pyrimidin-2-yl]amino]ethyl]phenyl] acetate | Intermediate IA1 and intermediate IA36 | 673.4 [MH⁺] |
| 47 | 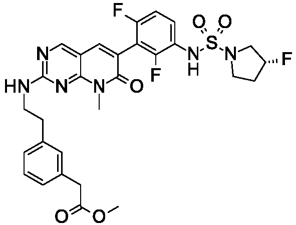 | methyl 2-[3-[2-[[6-[2,6-difluoro-3-[[(3R)-3-fluoropyrrolidin-1-yl]sulfonylamino]phenyl ]-8-methyl-7-oxopyrido[2,3-d]pyrimidin-2-yl]amino]ethyl]phenyl] acetate | Intermediate IA1 and intermediate IA35 | 631.3 [MH⁺] |
| 48 | 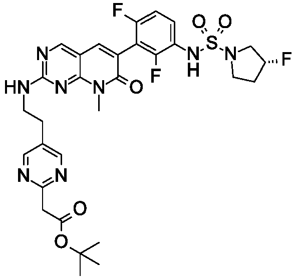 | tert-butyl 2-[5-[2-[[6-[2,6-difluoro-3-[[(3R)-3-fluoropyrrolidin-1-yl]sulfonylamino]phenyl ]-8-methyl-7-oxopyrido[2,3-d]pyrimidin-2-yl]amino]ethyl]pyrimidin -2-yl]acetate | Intermediate IA1 and intermediate IA37 | 673.4 [MH⁺] |

### Example 14

### (3R)-N-[2,4-difluoro-3-[8-methyl-7-oxo-2-(2-piperidin-4-ylethylamino)pyrido[2,3-d]pyrimidin-6-yl]phenyl]-3-fluoropyrrolidine-1-sulfonamide hydrochloride

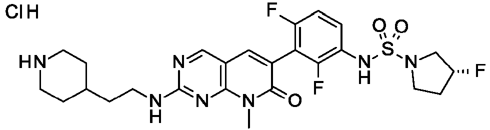

tert-Butyl (R)-4-(2-((6-(2,6-difluoro-3-((3-fluoropyrrolidine)-1-sulfonamido)phenyl)-8-methyl-7-oxo-7,8-dihydropyrido[2,3-d]pyrimidin-2-yl)amino)ethyl)piperidine-1-carboxylate (example 1, 155 mg, 233 µmol) was dissolved in 4M HCl in dioxane (2.5 ml, 10 mmol) and was then stirred at rt for 4 hours. After this time, LC-MS showed complete consumption of the starting material. The reaction mixture was concentrated *in vacuo* and was further dried under high vaccum. (R)-N-(2,4-Difluoro-3-(8-methyl-7-oxo-2-((2-(piperidin-4-yl)ethyl)amino)-7,8-dihydropyrido[2,3-d]pyrimidin-6-yl)phenyl)-3-fluoropyrrolidine-1-sulfonamide hydrochloride (160 mg, 226 µmol, 97 % yield) was obtained as a yellow crystalline solid. MS (ESI⁻): 564.4 [(M-HCl-H)⁻].

### Examples 15 - 19

The following examples were made in analogy to example 13, by using the appropriate intermediates and reagents:

| **Ex.** | **Structure** | **Name** | **Intermediate / Reagent** | **MS (ESI)** |
|---|---|---|---|---|
| 15 | 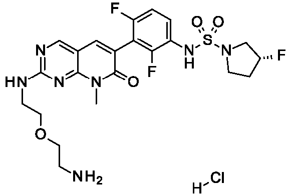 | (3R)-N-[3-[2-[2-(2-aminoethoxy)ethylamino ]-8-methyl-7-oxopyrido[2,3-d]pyrimidin-6-yl]-2,4-difluorophenyl]-3-fluoropyrrolidine-1-sulfonamide hydrochloride | Intermediate IA9 | 542.2 [(M-HCl+H ]⁺ |
| 16 | 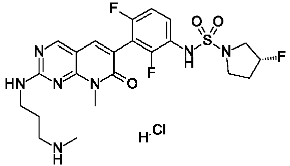 | (3R)-N-[2,4-difluoro-3-[8-methyl-2-[3-(methylamino)propylamino ]-7-oxopyrido[2,3-d]pyrimidin-6-yl]phenyl]-3-fluoropyrrolidine-1-sulfonamide hydrochloride | Intermediate IA10 | 524.4 [M-HCl+H ]⁺ |
| 17 | 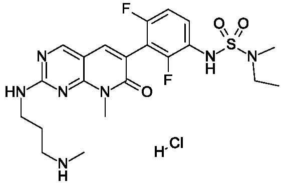 | 6-[3-[[ethyl(methyl)sulfamoyl ]amino]-2,6-difluorophenyl]-8-methyl-2-[3-(methylamino)propylamino ]-7-oxopyrido[2,3-d]pyrimidine hydrochloride | Intermediate IA11 | 496.3 [M-HCl+H ]⁺ |
| 18 | 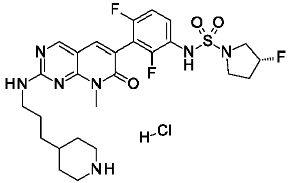 | (3R)-N-[2,4-difluoro-3-[8-methyl-7-oxo-2-(3-piperidin-4-ylpropylamino)pyrido[2 ,3-d]pyrimidin-6-yl]phenyl]-3-fluoropyrrolidine-1-sulfonamide hydrochloride | Intermediate IA12 | 578.4 [M-HCl-H]⁻ |
| 19 | 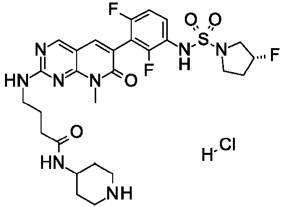 | 4-[[6-[2,6-difluoro-3-[[(3R)-3-fluoropyrrolidin-1-yl]sulfonylamino]pheny l]-8-methyl-7-oxopyrido[2,3-d]pyrimidin-2-yl]amino]-N-piperidin-4-ylbutanamide hydrochloride | Intermediate IA26 | 621.3 [M-HCl-H]⁻ |

### Example 20

### (3R)-N-[3-[2-[2-(1-acetylpiperidin-4-yl)ethylamino]-8-methyl-7-oxopyrido[2,3-d]pyrimidin-6-yl]-2,4-difluorophenyl]-3-fluoropyrrolidine-1-sulfonamide

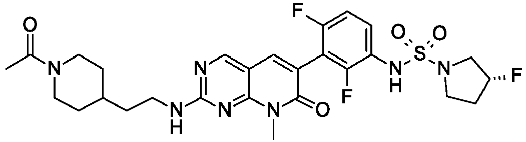

(R)-N-(2,4-difluoro-3-(8-methyl-7-oxo-2-((2-(piperidin-4-yl)ethyl)amino)-7,8-dihydropyrido[2,3-d]pyrimidin-6-yl)phenyl)-3-fluoropyrrolidine-1-sulfonamide hydrochloride (Example 2, 10 mg, 16.6 µmol) was dissolved in aqueous sodium bicarbonate solution (1M) (83 µl) and DCM (59µl) was added. Acetic anhydride (2.35 µl, 24.9 µmol) was added and the reaction mixture was stirred at rt. LC-MS confirmed completion of the reaction compete after addition of the acetic anhydride. The reaction mixture was diluted with 15 ml DCM and 15 ml water. The layers were separated, and the aqueous layer was extracted with 2 x 15 ml DCM. The combined organic layers were washed with 15 ml brine, dried with anhydrous sodium sulfate, filtered and concentrated to give 9.2 mg of a crude material. This material was dissolved in a minmal amount of DCM and the solution was applied to a 4 g column and was eluted with a gradient of 0 - 5% MeOH in DCM. The fractions containing pure product were combined and evaporated to dryness to give 6.8 mg (67%) of the desired material as a white and waxy solid. MS (ESI-): 606.4 [(M-H)⁻].

### Examples 21 - 33

The following examples were made in analogy to example 13, by using the appropriate intermediates and reagents:

| **Ex.** | **Structure** | **Name** | **Intermediate / Reagent** | **MS (ESI)** |
|---|---|---|---|---|
| 21 | 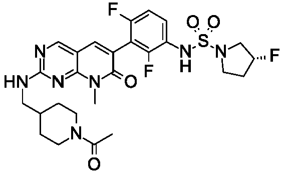 | (3R)-N-[3-[2-[(1-acetylpiperidin-4-yl)methylamino]-8-methyl-7-oxopyrido[2,3-d]pyrimidin-6-yl]-2,4-difluorophenyl]-3-fluoropyrrolidine-1-sulfonamide | Intermediate IA19 | 594.4 [MH⁺] |
| 22 | 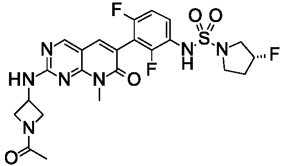 | (3R)-N-[3-[2-[(1-acetylazetidin-3-yl)amino]-8-methyl-7-oxopyrido[2,3-d]pyrimidin-6-yl]-2,4-difluorophenyl]-3-fluoropyrrolidine-1-sulfonamide | Intermediate IA18 | 552.2 [MH⁺] |
| 23 | 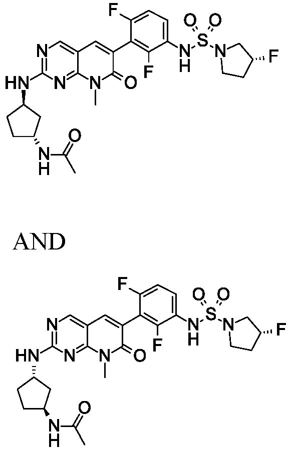 mixture of 2 diastereomers | N-[(1R,3R)-3-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]cyclopentyl]ace tamide AND N-[(1S,3S)-3-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]cyclopentyl]ace tamide | Intermediate IA20 | 580.2 [MH⁺] |
| 24 | 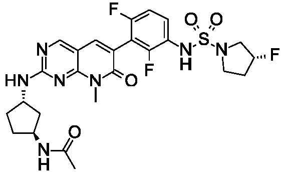 | N-[(1S,3S)-3-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]cyclopentyl] acetamide | Intermediate IA21 | 580.3 [MH⁺] |
| 25 | 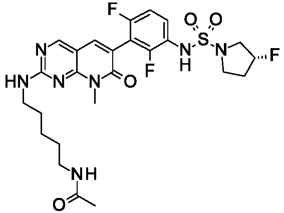 | N-[5-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]pentyl] acetamide | Intermediate IA22 | 582.3 [MH⁺] |
| 26 | 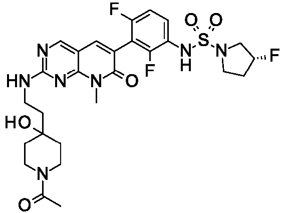 | (3R)-N-[3-[2-[2-(1-acetyl-4-hydroxypiperidin-4-yl)ethylamino]-8-methyl-7-oxopyrido[2,3-d]pyrimidin-6-yl]-2,4-difluorophenyl]-3-fluoropyrrolidine-1-sulfonamide | Intermediate IA23 | 624.4 [MH⁺] |
| 27 | 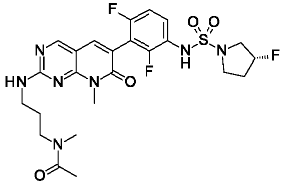 | N-[3-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]propyl]-N-methylacetamide | Example 16 | 568.2 [MH⁺] |
| 28 | 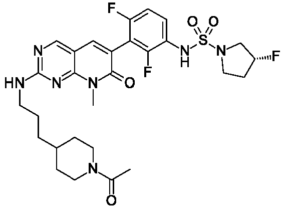 | (3R)-N-[3-[2-[3-(1-acetylpiperidin-4-yl)propylamino]-8-methyl-7-oxopyrido[2,3-d]pyrimidin-6-yl]-2,4-difluorophenyl]-3-fluoropyrrolidine-1-sulfonamide | Example 18 | 620.4 [M-H]⁻ |
| 29 | 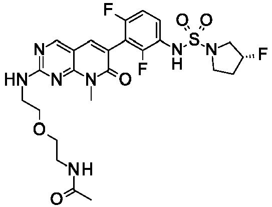 | N-[2-[2-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]ethoxy]ethyl] acetamide | Example 15 | 584.2 [MH⁺] |
| 30 | 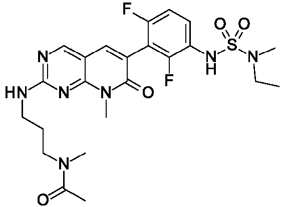 | N-[3-[[6-[3-[[ethyl(methyl)sulfamoyl ]amino]-2,6-difluorophenyl]-8-methyl-7-oxopyrido[2,3-d]pyrimidin-2-yl]amino]propyl]-N-methylacetamide | Example 17 | 538.3 [MH⁺] |
| 31 | 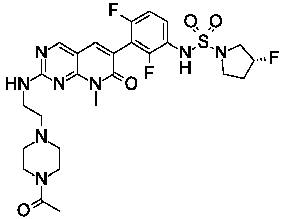 | (3R)-N-[3-[2-[2-(4-acetylpiperazin-1-yl)ethylamino]-8-methyl-7-oxopyrido[2,3-d]pyrimidin-6-yl]-2,4-difluorophenyl]-3-fluoropyrrolidine-1-sulfonamide | Intermediate IA24 | 609.5 [MH⁺] |
| 32 | 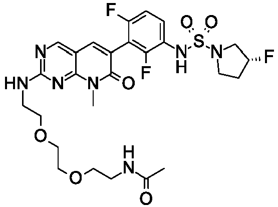 | N-[2-[2-[2-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]ethoxy]ethoxy] ethyl]acetamide | Intermediate IA25 | 628.4 [MH⁺] |
| 33 | 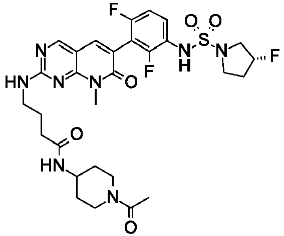 | N-(1-acetylpiperidin-4-yl)-4-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]butanamide | Example 19 | 665.2 [MH⁺] |

### Example 34

### N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide

### Step 1: (3R)-N-[3-[2-[2-[3-(aminomethyl)phenyl]ethylamino]-8-methyl-7-oxopyrido[2,3-d]pyrimidin-6-yl]-2,4-difluorophenyl]-3-fluoropyrrolidine-1-sulfonamide

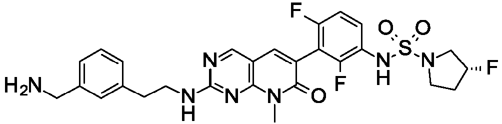

(R)-N-(3-(2-((3-cyanophenethyl)amino)-8-methyl-7-oxo-7,8-dihydropyrido[2,3-d]pyrimidin-6-yl)-2,4-difluorophenyl)-3-fluoropyrrolidine-1-sulfonamide (Intermediate IA27, 44 mg, 75.4 µmol) was dissolved in methanol (440 µl) and the solution was cooled to 0 C. Cobalt chloride (53.8 mg, 226 µmol) was added portionwise over 5 mins. Then, NaBH₄ (28.5 mg, 754 µmol) was added over 5 mins and the reaction mixture was warmed to rt. After 2 hours, the reaction did not progress further so more NaBH₄ (57 mg, 1.51 mmol) was added. Two additional crops of NaBH₄ (57 mg, 1.51 mmol) were added over 4 hours, and then the reaction was left over the weekend. Full conversion still not seen after this time so more NaBH₄ (57 mg, 1.51 mmol) was added. However, side products started to appear now and so the reaction was worked up. The reaction mixture was poured into 15 ml, 1M HCl and was washed with 15 ml DCM. The pH of the aqueous layer was adjusted to pH 8 by addition of saturated NaHCO₃ solution and then the aqueous solution was extracted with 4 x 15 ml DCM. The combined organic extracts were washed with 15 ml brine, dried, filtered and concentrated to give 5.5 mg of an impure product with about 60% purity, which was used in the next reaction step without further purification. MS (ESI⁻): 586.5 [(M-H)⁻].

### Step 2: N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide

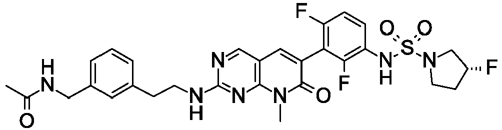

(R)-N-(3-(2-((3-(aminomethyl)phenethyl)amino)-8-methyl-7-oxo-7,8-dihydropyrido[2,3-d]pyrimidin-6-yl)-2,4-difluorophenyl)-3-fluoropyrrolidine-1-sulfonamide (5.5 mg, 9.36 µmol) obtained in the previous reaction step was dissolved in aqueous sodium bicarbonate solution (1M, 46.8 µl, 46.8 µmol) and DCM (33.4 µl). Acetic anhydride (2.65 µl, 28.1 µmol) was added and the reaction was showed to be complete after 15 minutes by LC-MS. The reaction mixture was diluted with 10 ml DCM and 10 ml water. The layers were separated and the aqueous layer extracted with additional 2 x 10 ml DCM. The combined organic layers were washed with 10 ml brine, dried with anhydrous sodium sulfate, filtered and concentrated in vacuo to give 4.9 mg of a crude product. This material was dissolved in a minimal amount of DCM, applied to a 4 g column and eluted with a gradient of 0 - 5% MeOH in DCM. Product fractions were combined and concentrated to give 2.4 mg (41%) of the title compound as a white solid. MS (ESI): 630.4 [MH⁺].

### Example 35

The following example was made in analogy to example 34, steps 1 and 2, by using the appropriate intermediates as starting materials and the corresponding reagents for the reaction steps:

| **Ex.** | **Structure** | **Name** | **Intermediate / Reagent** | **MS (ESI)** |
|---|---|---|---|---|
| 35 | 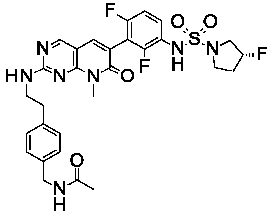 | N-[[4-[2-[[6-[2,6-difluoro-3-[[(3R)-3-fluoropyrrolidin-1-yl]sulfonylamino]phenyl] -8-methyl-7-oxopyrido[2,3-d]pyrimidin-2-yl]amino]ethyl]phenyl] methyl]acetamide | Intermediate IA28 used in step 1 | 630.5 [MH⁺] |

### Example 36

### N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]-N-methylacetamide

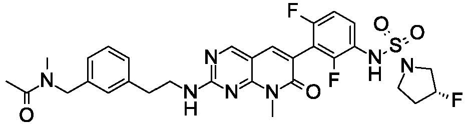

To a solution of tert-butyl (R)-(3-(2-((6-(2,6-difluoro-3-((3-fluoropyrrolidine)-1-sulfonamido)phenyl)-8-methyl-7-oxo-7,8-dihydropyrido[2,3-d]pyrimidin-2-yl)amino)ethyl)benzyl)(methyl)carbamate (Intermediate IA29, 13.5 mg, 19.2 µmol) in dioxane (300 µL) was added 4M HCl in dioxane (300 µl, 1.2 mmol). The reaction was stirred at rt. Complete conversion was observed after 20hrs. The reaction mixture was concentrated *in vacuo.*

The crude material was dissolved in DCM (300 µl). Triethylamine (5.4 µl, 38.5 µmol) and acetic anhydride (2 µl, 21.2 µmol) were added and the reaction was stirred at rt for 1h. LC-MS shows completion of the reaction. The reaction mixture was partitioned between EtOAc (15 mL) and sat. ammonium chloride solution (15 mL). The organic layer was separated and the aqueous layer was extracted with more EtOAc (3x15 mL). The combined organic layers were washed with aqueous citric acid solution (30 mL), sat. sodium bicarbonate solution (30 mL), water (30 mL) and brine (30 mL). The combined organic layers were dried over magnesium sulfate, filtered and concentrated *in vacuo.* This yielded the title compound as a light brown solid (15.7 mg). MS (ESI): 644.4 [MH⁺].

### Example 37

The following example was made in analogy to example 36 by using the appropriate intermediates as starting materials and the corresponding reagents for the reaction steps:

| **Ex.** | **Structure** | **Name** | **Intermediate / Reagent** | **MS (ESI)** |
|---|---|---|---|---|
| 37 | 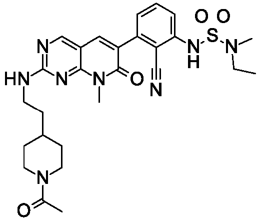 | 2-[2-(1-acetylpiperidin-4-yl)ethylamino]-6-[2-cyano-3-[[ethyl(methyl)sulfamoyl ]amino]phenyl]-8-methyl-7-oxopyrido[2,3-d]pyrimidine | Intermediate IA30 | 567.4 [MH⁺] |

### Example 38

### tert-Butyl (3RS)-3-[2-(cyclopropylmethylamino)-6-[2,6-difluoro-3-[[(3R)-3-fluoropyrrolidin-1-yl]sulfonylamino]phenyl]-7-oxopyrido[2,3-d]pyrimidin-8-yl]pyrrolidine-1-carboxylate

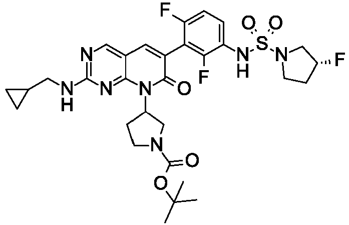

This compound was made in analogy to the synthesis of Intermediate IA31, steps 1 - 7, by using the appropriate amine tert-butyl 3-aminopyrrolidine-1-carboxylate as a starting material in reaction step 1. Following this reaction sequence, the title compound was obtained as an off-white solid (30 mg). MS (ESI): 664.3 [MH⁺].

### Example 39

### (3R)-N-[3-[8-[2-[2-(2-aminoethoxy)ethoxy]ethyl]-2-(cyclopropylmethylamino)-7-oxopyrido[2,3-d]pyrimidin-6-yl]-2,4-difluorophenyl]-3-fluoropyrrolidine-1-sulfonamide; hydrochloride

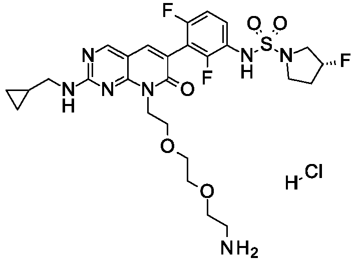

tert-Butyl (R)-(2-(2-(2-(2-((cyclopropylmethyl)amino)-6-(2,6-difluoro-3-((3-fluoropyrrolidine)-1-sulfonamido)phenyl)-7-oxopyrido[2,3-d]pyrimidin-8(7H)-yl)ethoxy)ethoxy)ethyl)carbamate (Intermediate IA31, 56 mg, 77.2 µmol) was dissolved in dioxane (340 µl) and 4M HCl in dioxane (289 µl, 1.16 mmol) was added and the mixture was stirred at rt for 3.5 hours. Then, the reaction mixture was concentrated *in vacuo* to afford 48.4 mg (95%) of the title compound as a pale yellow foam. MS (ESI): 624.4 [(M-HCl-H)⁻].

### Example 40

The following example was made in analogy to example 38 by using the appropriate starting materials and reagents:

| **Ex.** | **Structure** | **Name** | **Starting material** | **MS (ESI)** |
|---|---|---|---|---|
| 40 | 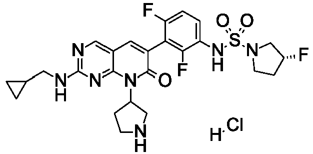 | (3R)-N-[3-[2-(cyclopropylmethylam ino)-7-oxo-8-[(3RS)-pyrrolidin-3-yl]pyrido[2,3-d]pyrimidin-6-yl]-2,4-difluorophenyl]-3-fluoropyrrolidine-1-sulfonamide hydrochloride | Example 38 | 564.2 [(M-HCl+H)⁺] |

### Example 41

### N-[2-[2-[2-[2-(cyclopropylmethylamino)-6-[2,6-difluoro-3-[[(3R)-3-fluoropyrrolidin-1-yl]sulfonylamino]phenyl]-7-oxopyrido[2,3-d]pyrimidin-8-yl]ethoxy]ethoxy]ethyl]acetamide

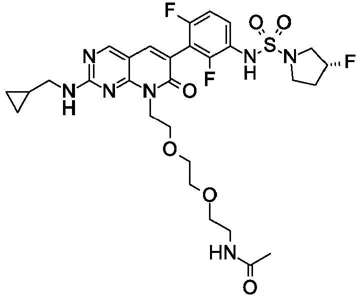

(R)-N-(3-(8-(2-(2-(2-Aminoethoxy)ethoxy)ethyl)-2-((cyclopropylmethyl)amino)-7-oxo-7,8-dihydropyrido[2,3-d]pyrimidin-6-yl)-2,4-difluorophenyl)-3-fluoropyrrolidine-1-sulfonamide hydrochloride (Example 39, 15 mg, 22.7 µmol) was dissolved in aqueous 1M sodium bicarbonate solution (113 µl, 113 µmol) and DCM (81 µl). Acetic anhydride (6.41 µl, 68 µmol) was added and the reaction was shown to be complete immediately after addition. The reaction mixture was diluted with 15 ml DCM and 15 ml water. The layers were separated and the aqueous layer was extracted with 2 x 15 ml DCM. The combined organic layers were washed with 15 ml brine, dried with anhydrous Na₂SO₄, filtered and evaporated to give 13 mg of a crude product. This material was dissolved in a minimal amount of DCM and was applied to a 4 g column, followed by elution with a gradient of 0 - 5% MeOH in DCM. Product fractions were combined and concentrated *in vacuo* to afford 10.2 mg (67%) of the title compound as a white solid. MS (ESI): 668.4 [MH⁺].

### Examples 42 and 43

The following examples were made in analogy to example 38 by using the appropriate starting materials and the corresponding reagents:

| **Ex.** | **Structure** | **Name** | **Starting material** | **MS (ESI)** |
|---|---|---|---|---|
| 42 | 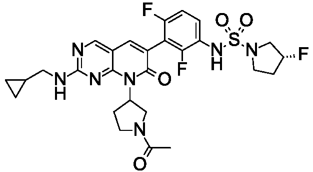 | (3R)-N-[3-[8-((3RS)-1-acetylpyrrolidin-3-yl)-2-(cyclopropylmethylamin o)-7-oxopyrido[2,3-d]pyrimidin-6-yl]-2,4-difluorophenyl]-3-fluoropyrrolidine-1-sulfonamide | Example 40 | 606.3 [MH⁺] |
| 43 | 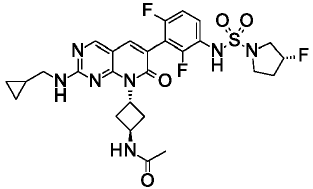 | trans-N-[3-[2-(cyclopropylmethylamin o)-6-[2,6-difluoro-3-[[(3R)-3-fluoropyrrolidin-1-yl]sulfonylamino]phenyl] -7-oxopyrido[2,3-d]pyrimidin-8-yl]cyclobutyl]acetamide | Intermedia te IA33 | 606.4 [MH⁺] |

### Example 44

### 2-(Cyclopropylmethylamino)-6-[3-[[ethyl(methyl)sulfamoyl]amino]-2,6-difluorophenyl]-7-oxo-8-(2-phenylmethoxyethyl)pyrido[2,3-d]pyrimidine

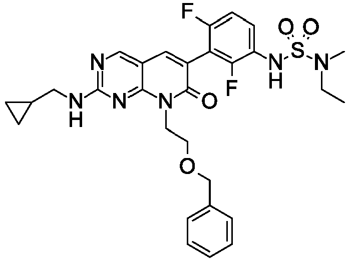

This material was made in analogy to Intermediate IA31, steps 1 -7, by using the corresponding reagents and building blocks in the appropriate reaction steps: 2-phenylmethoxyethanamine in step 1 to introduce the 2-benzyloxyethyl side chain; and N-ethyl-N-methylsulfamoyl chloride in step 5 to introduce the ethyl(methyl)sulfamoyl moiety. With this reaction sequence, the title compound was obtained as a colorless solid: MS (ESI): 599.2 [MH⁺].

### Example 49

### (3R)-N-[3-[2-[2-(2-Acetyl-1,3-dihydroisoindol-5-yl)ethylamino]-8-methyl-7-oxopyrido[2,3-d]pyrimidin-6-yl]-2,4-difluorophenyl]-3-fluoropyrrolidine-1-sulfonamide

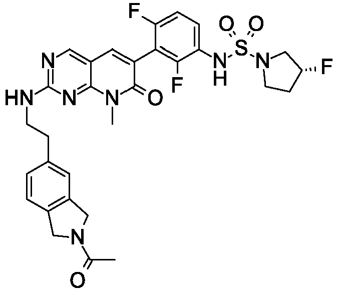

To a solution of (R)-N-(2,4-difluoro-3-(2-((2-(isoindolin-5-yl)ethyl)amino)-8-methyl-7-oxo-7,8-dihydropyrido[2,3-d]pyrimidin-6-yl)phenyl)-3-fluoropyrrolidine-1-sulfonamide hydrochloride (intermediate IA38, 10 mg, 15.7 µmol) in DCM (200 µL) was added NEt₃ (4.4 µL, 31.4 µmol) and acetic anhydride (2 µL, 21.2 µmol). The reaction was stirred at rt for 2h. Then, the reaction mixture was partitioned between EtOAc (5 ml) and an NH₄Cl solution (sat. aq., 5 mL). The organic layer was separated and the aqueous layer was extracted with more EtOAc (2x5 mL). The combined organic layers were washed with a citric acid solution (10% aq., 10 mL), a sodium bicarbonate solution (sat. aq., 10 mL), water (10 mL) and brine (10 mL). The combined organic layers were dried over magnesium sulfate, filtered and concentrated *in vacuo* to provide the title compound as a light brown solid (6.8 mg, 60%, purity approx. 89% (UV)). MS (ESI): 642.4 [MH⁺].

### Example 50

The following example was made in analogy to example 49 by using the appropriate starting materials and reagents:

| **Ex.** | **Structure** | **Name** | **Starting material** | **MS (ESI)** |
|---|---|---|---|---|
| 50 | 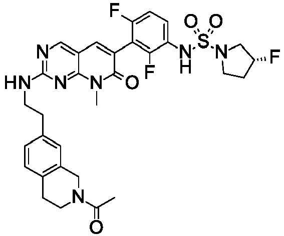 | (3R)-N-[3-[2-[2-(2-acetyl-3,4-dihydro-1H-isoquinolin-7-yl)ethylamino]-8-methyl-7-oxopyrido[2,3-d]pyrimidin-6-yl]-2,4-difluorophenyl]-3-fluoropyrrolidine-1-sulfonamide | Intermediate IA41 | 656.4 [MH⁺] |

### Example 51

### Materials

### Cell lines

A375 is a cellular cancer model expressing V600E mutated BRAF and HCT116 a cellular cancer model expressing WT BRAF. First generation BRAF inhibitors such as e.g. dabrafenib induce a paradox effect on tumour cells in that they inhibit the growth of V600E mutated BRAF cells (such as e.g. A375), while they activate growth in WT BRAF cells (such as e.g. HCT 116). ERK 1,2 phosphorylation (terminal member of the phosphorylation cascade of the MAPK pathway) is hereafter reported as main readout for the activation status of the MAPK pathway. DMEM no-phenol red medium supplemented with L-glutamine was purchased from (Thermo Fisher Scientific). Fetal bovine serum (FBS) was purchased from VWR. Advanced ERK phospho-T202 /Y204 kit - 10,000 tests was purchased from Cisbio cat# 64AERPEH. A375 (catalog# CRL-1619) and HCT116 (catalog# CCL-247) cells were originally obtained from American type Culture Collection (ATCC) and banked by the Roche repository. 384-Well microplates ultized were purchased from Greiner Bio-One, 384-well, (With Lid, HiBase, Low volume cat 784-080).

### HTRF assay for P-ERK determination in A375 or HCT116 cells

Prior to the assay, A375 and HCT116 cell lines are maintained in DMEM no-phenol red medium supplemented with 10% fetal bovine serum (FBS). Following compound treatment, P-ERK levels are determined by measuring FRET fluorescence signal induced by selective binding of two antibodies provided in the mentioned kit (Cisbio cat# 64AERPEH) on ERK protein when phosphorylated at Thr202/Tyr204. Briefly, 8000 cells/well in 12 µl media/well are plated in the 384-well plate and left overnight in the incubator (at 37 °C with 5% CO₂-humidified atmosphere), the following day the plate is treated in duplicate with test compounds, dabrafenib and PLX8394 (the latter two as controls) at the following final drug concentrations: 10µM, 3µM, 1µM, 0.3µM, 0.1µM, 0.03µM, 0,01µM, 0.003µM and 0.001µM. All wells are subjected to DMSO normalization and drug incubation occurs for 1 hour. Then, 4µl of a 4X lysis buffer supplied with the kit are added to the wells, the plate is then centrifuged for 30 second (3000rpm) and incubated on a plate shaker for 1h at rt.

At the end of the incubation 4µl/well of advanced P-ERK antibody solution (prepared according to manufacturer's instruction) followed by 4µl/well of criptate P-ERK antibody solution (prepared according to manufacturer's instruction) (Cisbio cat# 64AERPEH) are added to test wells.

In order to allow proper data normalization, control wells non drug treated reported in the following table are always included in each plate (according to manufacturer's instruction):
p-ERK HTRF well compositions (µl):

| neg ctrl | pos ctrl | neut ctrl | cpd | blank | |
|---|---|---|---|---|---|
| - | - | 12 | 12 | 12 | Cells |
| 12 | - | - | - | - | Media |
| - | - | - | <0.05 | - | Cpd |
| - | 16 | - | - | - | control lysate (ready-to-use) |
| 4 | - | 4 | 4 | 4 | 4x lysis buffer |
| 4 | 4 | 4 | 4 | - | Advanced p-ERK antibody solution |
| - | - | - | - | 4 | Advanced p-ERK1/2 Cryptate antibody solut. |
| 20 | 20 | 20 | 20 | 20 | Total volume in Well |

The plate is then centrifuged at 3000 rpm for 30 seconds, sealed to prevent evaporation and incubated overnight in the dark at room temperature. The plate is then analyzed and fluorescence emission value collected through a Pherastast FSX (BMG Labtech) apparatus at 665 and 620 nM. The obtained fluorescence values are processed according to the formula Ratio=Signal(620nm)/Signal(625nm)*10000 then the average of the ratio on the blank is subtracted to all values.

Data are normalized in the case of A375 cells (BRAF inhibition) considering the average of the ratio (blank subtracted) derived by DMSO only treated cells as 100% and by considering the average of the ratio (blank subtracted) derived by 10 µM Dabrafenib treated cells as 0%. Mean of the normalized points are fitted with sigmoidal curve and IC₅₀ values are determined.

Data are normalized in the case of HCT116 cells (BRAF activation) considering the average of the ratio (blank subtracted) derived by DMSO only treated cells as 0% and by considering the average of the ratio (blank subtracted) derived by Dabrafenib treated cells at the concentration which provides the highest signal as 100%. Individual points are fitted with either sigmoidal or bell shape curves, the percentage of activation compared to maximum Dabrafenib-mediated activation is determined. The results of the assay are shown below in Table 1.

**Table 1**

| **Ex.** | **K_{D} BRAF V660E [µM]** | **K_{D} BRAF [µM]** | **P-ERK_A375: IC₅₀ (µM)** | **P-ERK_HCT-116: EC_{50_50PCT} (µM)** | **Fold Selectivity HCT-116 EC_{50- 50} / A375** |
|---|---|---|---|---|---|
| **1** | 0.1899 | 0.1082 | 0.0633 | 3.3622 | 53.1 |
| **2** | 0.0230 | 0.0079 | 0.0060 | 0.4912 | 81.8 |
| **3** | 0.0474 | 0.0151 | 0.0131 | 0.9169 | 69.8 |
| **4** | 0.0990 | 0.0399 | 0.0474 | 10.0000 | 211.0 |
| **5** | 0.1149 | 0.0465 | 0.0426 | 0.3941 | 9.2 |
| **6** | 0.1197 | 0.0357 | 0.0134 | 0.7110 | 52.9 |
| **7** | 0.2822 | 0.0912 | 0.0308 | 2.1710 | 70.5 |
| **8** | 0.3138 | 0.2615 | 0.2413 | 9.7635 | 40.5 |
| **9** | 0.4169 | 0.1359 | 0.1529 | 10.0000 | 65.4 |
| **10** | 0.0375 | 0.0190 | 0.0099 | 0.6454 | 65.1 |
| **11** | 0.4409 | 0.1585 | 0.0710 | 10.0000 | 140.9 |
| **12** | 0.4868 | 0.0849 | 0.1088 | 2.2212 | 20.4 |
| **13** | 0.4402 | 0.3496 | 0.2723 | 9.4616 | 34.7 |
| **14** | 0.1067 | 0.0377 | 0.7904 | 10.0000 | 12.7 |
| **15** | 0.1944 | 0.0817 | 0.6168 | 10.0000 | 16.2 |
| **16** | 0.2962 | 0.1483 | 0.6872 | 10.0000 | 14.6 |
| **17** | 0.3849 | 0.1676 | 1.2646 | 7.5250 | 6.0 |
| **18** | 0.0716 | 0.0255 | 0.2072 | 5.8692 | 28.3 |
| **19** | 0.0596 | 0.0383 | 7.2124 | 10.0000 | 1.4 |
| **20** | 0.0270 | 0.0102 | 0.0367 | 1.6492 | 44.9 |
| **21** | 0.1093 | 0.0620 | 0.0867 | 10.0000 | 115.3 |
| **22** | 0.0208 | 0.0071 | 0.1837 | 10.0000 | 54.4 |
| **23** | 0.0211 | 0.0075 | 0.0690 | 4.0989 | 59.4 |
| **24** | 0.0275 | 0.0082 | 0.1255 | 8.8704 | 70.7 |
| **25** | 0.0268 | 0.0122 | 0.0440 | 3.1773 | 72.3 |
| **26** | 0.0275 | 0.0094 | 0.2707 | 10.0000 | 36.9 |
| **27** | 0.0419 | 0.0096 | 0.0548 | 1.8319 | 33.4 |
| **28** | 0.0530 | 0.0147 | 0.0334 | 1.3664 | 40.9 |
| **29** | 0.0601 | 0.0201 | 0.3750 | 10.0000 | 26.7 |
| **30** | 0.1590 | 0.0723 | 0.2873 | 5.6968 | 19.8 |
| **31** | 0.0522 | 0.0210 | 0.0798 | 6.4029 | 80.2 |
| **32** | 0.0700 | 0.0236 | 0.1955 | 10.0000 | 51.1 |
| **33** | 0.0601 | 0.0249 | 3.1710 | 10.0000 | 3.2 |
| **34** | 0.0040 | 0.0018 | 0.0115 | 0.6806 | 59.1 |
| **35** | 0.0221 | 0.0057 | 0.0373 | 1.4921 | 40.0 |
| **36** | 0.0172 | 0.0089 | 0.0205 | 2.7275 | 133.4 |
| **37** | 0.0040 | 0.0022 | 0.0495 | 0.4634 | 9.4 |
| **38** | 2.8228 | 1.4204 | 0.8258 | 10.0000 | 12.1 |
| **39** | 0.0413 | 0.0134 | 10.0000 | 10.0000 | 1.0 |
| **40** | 0.4075 | 0.1446 | 3.8088 | 10.0000 | 2.6 |
| **41** | 0.0561 | 0.0212 | 0.8442 | 10.0000 | 11.8 |
| **42** | 0.0504 | 0.0226 | 0.3776 | 4.8352 | 12.8 |
| **43** | 0.0129 | 0.0055 | 0.1078 | 0.4037 | 3.7 |
| **44** | 1.7466 | 1.3801 | 1.9022 | 10.0000 | 5.3 |
| **45** | na | na | 0.0268 | 1.4772 | 55.1 |
| **46** | 0.2235 | 0.0967 | 0.0642 | 3.5308 | 55.0 |
| **47** | 0.0232 | 0.0092 | 0.0385 | 4.4655 | 116.0 |
| **48** | 0.0566 | 0.0266 | 0.1757 | 5.9152 | 33.7 |
| **49** | 0.0065 | 0.0025 | 0.0084 | 0.9891 | 117.8 |
| **50** | 0.0138 | 0.0045 | 0.015 | 1.554 | 103.6 |

### Example A

Film coated tablets containing the following ingredients can be manufactured in a conventional manner:

| Ingredients | Per tablet | |
|---|---|---|
| **Kernel:** | | |
| Compound of formula (I) | 10.0 mg | 200.0 mg |
| Microcrystalline cellulose | 23.5 mg | 43.5 mg |
| Lactose hydrous | 60.0 mg | 70.0 mg |
| Povidone K30 | 12.5 mg | 15.0 mg |
| Sodium starch glycolate | 12.5 mg | 17.0 mg |
| Magnesium stearate | 1.5 mg | 4.5 mg |
| (Kernel Weight) | 120.0 mg | 350.0 mg |

| **Film Coat:** | | |
|---|---|---|
| Hydroxypropyl methyl cellulose | 3.5 mg | 7.0 mg |
| Polyethylene glycol 6000 | 0.8 mg | 1.6 mg |
| Talc | 1.3 mg | 2.6 mg |
| Iron oxide (yellow) | 0.8 mg | 1.6 mg |
| Titan dioxide | 0.8 mg | 1.6 mg |

The active ingredient is sieved and mixed with microcrystalline cellulose and the mixture is granulated with a solution of polyvinylpyrrolidone in water. The granulate is then mixed with sodium starch glycolate and magnesium stearate and compressed to yield kernels of 120 or 350 mg respectively. The kernels are lacquered with an aq. solution / suspension of the above mentioned film coat.

### Example B

Capsules containing the following ingredients can be manufactured in a conventional manner:

| Ingredients | Per capsule |
|---|---|
| Compound of formula (I) | 25.0 mg |
| Lactose | 150.0 mg |
| Maize starch | 20.0 mg |
| Talc | 5.0 mg |

The components are sieved and mixed and filled into capsules of size 2.

### Example C

Injection solutions can have the following composition:

| | |
|---|---|
| Compound of formula (I) | 3.0 mg |
| Polyethylene glycol 400 | 150.0 mg |
| Acetic acid | q.s. ad pH 5.0 |
| Water for injection solutions | ad 1.0 ml |

The active ingredient is dissolved in a mixture of Polyethylene glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

## Claims

1. A compound of formula (I) 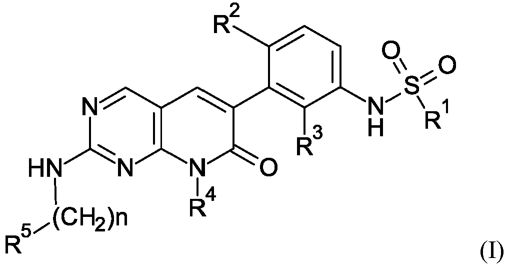 wherein
R¹ is dialkylamino, (cycloalkyl)(alkyl)amino, haloheterocycloalkyl, heterocycloalkyl or cycloalkyl;
R² and R³ are independently selected from hydrogen, cyano and halogen;
R⁴ is alkyl, alkoxycarbonylheterocycloalkyl, aminoalkoxyalkoxyalkyl, heterocycloalkyl, alkylcarbonylaminoalkoxyalkoxyalkyl, alkylcarbonylheterocycloalkyl, alkylcarbonylaminocycloalkyl or phenylalkoxyalkyl;
n = 0, 1, 2, 3, 4 or 5; and
R⁵ is alkoxycarbonyl, alkoxycarbonylalkylphenyl, alkoxycarbonylalkylheterocycloalkyl, alkoxycarbonylheterocycloalkyl, alkylamino, alkylcarbonyl(alkylamino), alkylcarbonyl(alkylamino)alkylphenyl, alkylcarbonylamino, alkylcarbonylaminoalkoxy, alkylcarbonylaminoalkoxyalkoxy, alkylcarbonylheterocycloalkylaminocarbonyl, alkylcarbonylaminoalkylphenyl, alkylcarbonyl(hydroxyheterocycloalkyl), alkylcarbonylheterocycloalkyl, alkylcarbonylaminocycloalkyl, aminoalkoxy, cycloalkyl, heterocycloalkyl, heterocycloalkylaminocarbonyl, phenyloxy, hydroxyalkoxyalkoxy, phenyl or hydrogen;
or a pharmaceutically acceptable salt thereof.

2. A compound according to claim 1, wherein R¹ is dialkylamino, (cycloalkyl)(alkyl)amino, halopyrrolidinyl, pyrrolidinyl or cycloalkyl.

3. A compound according to claim 1 or 2, wherein R¹ is (ethyl)(methyl)amino, (cyclopropyl)(methyl)amino, fluoropyrrolidinyl, pyrrolidinyl or cyclopentyl.

4. A compound according to any one of claims 1 to 3, wherein R¹ is dialkylamino or haloheterocycloalkyl.

5. A compound according to any one of claims 1 to 4, wherein R² and R³ are independently selected from hydrogen, cyano and fluorine.

6. A compound according to any one of claims 1 to 5, wherein R⁴ is methyl, tert-butyloxycarbonylpyrrolidinyl, aminoethoxyethoxyethyl, pyrrolidinyl, methylcarbonylaminoethoxyethoxyethyl, methylcarbonylpyrrolidinyl, methylcarbonylaminocyclobutyl or phenylmethoxyethyl.

7. A compound according to any one of claims 1 to 6, wherein R⁴ is alkyl.

8. A compound according to any one of claims 1 to 7, wherein n = 0, 1, 2, 3 or 5.

9. A compound according to any one of claims 1 to 8, wherein R⁵ is tert-butyloxycarbonyl, tert-butyloxycarbonylmethylphenyl, methoxycarbonylmethylphenyl, tert-butyloxycarbonylpiperidinyl, tert-butyloxycarbonylpiperazinyl, methylamino, methylcarbonyl(methylamino), methylcarbonyl(methylamino)methylphenyl, methylcarbonylamino, methylcarbonylaminoethoxyethoxy, methylcarbonylpiperidinylaminocarbonyl, methylcarbonylaminomethylphenyl, methylcarbonyl(hydroxypiperidinyl), methylcarbonylpiperidinyl, methylcarbonylpiperazinyl, methylcarbonylpiperidinyl, methylcarbonyl-1,3-dihydroisoindolyl, methylcarbonyl-1H-isoquinolinyl, aminoethoxy, cyclopropyl, cyclopentyl, piperidinyl, piperidinylaminocarbonyl, phenyloxy, hydroxyethoxyethoxy, phenyl or hydrogen.

10. A compound according to any one of claims 1 to 9, wherein R⁵ is phenyl, alkoxycarbonyl, cycloalkyl, alkylcarbonylpiperidinyl, alkylcarbonylpiperazinyl, alkylcarbonylamino, alkylcarbonyl(alkylamino), alkylcarbonylaminoalkylphenyl or alkylcarbonyl(alkylamino)alkylphenyl.

11. A compound according to any one of claims 1 to 10 selected from
tert-butyl 4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]piperidine-1-carboxylate;
(3R)-N-[2,4-difluoro-3-[8-methyl-7-oxo-2-(2-phenylethylamino)pyrido[2,3-d]pyrimidin-6-yl]phenyl]-3-fluoropyrrolidine-1-sulfonamide;
tert-butyl 4-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]butanoate;
(3R)-N-[2,4-difluoro-3-[2-[2-[2-(2-hydroxyethoxy)ethoxy]ethylamino]-8-methyl-7-oxopyrido[2,3-d]pyrimidin-6-yl]phenyl]-3-fluoropyrrolidine-1-sulfonamide;
(3R)-N-[2,4-difluoro-3-[8-methyl-7-oxo-2-(2-phenoxyethylamino)pyrido[2,3-d]pyrimidin-6-yl]phenyl]-3-fluoropyrrolidine-1-sulfonamide;
(3R)-N-[3-[2-(cyclopentylamino)-8-methyl-7-oxopyrido[2,3-d]pyrimidin-6-yl]-2,4-difluorophenyl]-3-fluoropyrrolidine-1-sulfonamide;
tert-butyl 4-[2-[[6-[2,6-difluoro-3-(pyrrolidin-1-ylsulfonylamino)phenyl]-8-methyl-7-oxopyrido[2,3-d]pyrimidin-2-yl]amino]ethyl]piperidine-1-carboxylate;
6-[3-[[ethyl(methyl)sulfamoyl]amino]-2,6-difluorophenyl]-2-[2-[2-(2-hydroxyethoxy)ethoxy]ethylamino]-8-methyl-7-oxopyrido[2,3-d]pyrimidine;
(3R)-N-[3-[2-(cyclopropylmethylamino)-8-methyl-7-oxopyrido[2,3-d]pyrimidin-6-yl]-2,4-difluorophenyl]-3-fluoropyrrolidine-1-sulfonamide;
2-(cyclopropylmethylamino)-6-[3-[[ethyl(methyl)sulfamoyl]amino]-2,6-difluorophenyl]-8-methyl-7-oxopyrido[2,3-d]pyrimidine;
2-(cyclopropylmethylamino)-6-[3-[[cyclopropyl(methyl)sulfamoyl]amino]-2,6-difluorophenyl]-8-methyl-7-oxopyrido[2,3-d]pyrimidine;
tert-butyl 4-[2-[[6-[3-(cyclopentylsulfonylamino)-2,6-difluorophenyl]-8-methyl-7-oxopyrido[2,3-d]pyrimidin-2-yl]amino]ethyl]piperidine-1-carboxylate;
(3R)-N-[2,4-difluoro-3-[8-methyl-7-oxo-2-(2-piperidin-4-ylethylamino)pyrido[2,3-d]pyrimidin-6-yl]phenyl]-3-fluoropyrrolidine-1-sulfonamide hydrochloride;
(3R)-N-[3-[2-[2-(2-aminoethoxy)ethylamino]-8-methyl-7-oxopyrido[2,3-d]pyrimidin-6-yl]-2,4-difluorophenyl]-3-fluoropyrrolidine-1-sulfonamide hydrochloride;
(3R)-N-[2,4-difluoro-3-[8-methyl-2-[3-(methylamino)propylamino]-7-oxopyrido[2,3-d]pyrimidin-6-yl]phenyl]-3-fluoropyrrolidine-1-sulfonamide hydrochloride;
(3R)-N-[2,4-difluoro-3-[8-methyl-7-oxo-2-(3-piperidin-4-ylpropylamino)pyrido[2,3-d]pyrimidin-6-yl]phenyl]-3-fluoropyrrolidine-1-sulfonamide hydrochloride;
(3R)-N-[3-[2-[2-(1-acetylpiperidin-4-yl)ethylamino]-8-methyl-7-oxopyrido[2,3-d]pyrimidin-6-yl]-2,4-difluorophenyl]-3-fluoropyrrolidine-1-sulfonamide;
(3R)-N-[3-[2-[(1-acetylpiperidin-4-yl)methylamino]-8-methyl-7-oxopyrido[2,3-d]pyrimidin-6-yl]-2,4-difluorophenyl]-3-fluoropyrrolidine-1-sulfonamide;
(3R)-N-[3-[2-[(1-acetylazetidin-3-yl)amino]-8-methyl-7-oxopyrido[2,3-d]pyrimidin-6-yl]-2,4-difluorophenyl]-3-fluoropyrrolidine-1-sulfonamide;
N-[(1R,3R)-3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]cyclopentyl]acetamide;
N-[(1S,3S)-3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]cyclopentyl]acetamide;
N-[5-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]pentyl]acetamide;
(3R)-N-[3-[2-[2-(1-acetyl-4-hydroxypiperidin-4-yl)ethylamino]-8-methyl-7-oxopyrido[2,3-d]pyrimidin-6-yl]-2,4-difluorophenyl]-3-fluoropyrrolidine-1-sulfonamide;
N-[3-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]propyl]-N-methylacetamide;
(3R)-N-[3-[2-[3-(1-acetylpiperidin-4-yl)propylamino]-8-methyl-7-oxopyrido[2,3-d]pyrimidin-6-yl]-2,4-difluorophenyl]-3-fluoropyrrolidine-1-sulfonamide;
N-[2-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethoxy]ethyl]acetamide;
N-[3-[[6-[3-[[ethyl(methyl)sulfamoyl]amino]-2,6-difluorophenyl]-8-methyl-7-oxopyrido[2,3-d]pyrimidin-2-yl]amino]propyl]-N-methylacetamide;
(3R)-N-[3-[2-[2-(4-acetylpiperazin-1-yl)ethylamino]-8-methyl-7-oxopyrido[2,3-d]pyrimidin-6-yl]-2,4-difluorophenyl]-3-fluoropyrrolidine-1-sulfonamide;
N-[2-[2-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethoxy]ethoxy]ethyl]acetamide;
N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide;
N-[[4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]acetamide;
N-[[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]methyl]-N-methylacetamide;
2-[2-(1-acetylpiperidin-4-yl)ethylamino]-6-[2-cyano-3-[[ethyl(methyl)sulfamoyl]amino]phenyl]-8-methyl-7-oxopyrido[2,3-d]pyrimidine;
tert-butyl (3RS)-3-[2-(cyclopropylmethylamino)-6-[2,6-difluoro-3-[[(3R)-3-fluoropyrrolidin-1-yl]sulfonylamino]phenyl]-7-oxopyrido[2,3-d]pyrimidin-8-yl]pyrrolidine-1-carboxylate;
N-[2-[2-[2-[2-(cyclopropylmethylamino)-6-[2,6-difluoro-3-[[(3R)-3-fluoropyrrolidin-1-yl]sulfonylamino]phenyl]-7-oxopyrido[2,3-d]pyrimidin-8-yl]ethoxy]ethoxy]ethyl]acetamide;
(3R)-N-[3-[8-((3RS)-1-acetylpyrrolidin-3-yl)-2-(cyclopropylmethylamino)-7-oxopyrido[2,3-d]pyrimidin-6-yl]-2,4-difluorophenyl]-3-fluoropyrrolidine-1-sulfonamide;
tert-butyl 4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]piperazine-1-carboxylate;
tert-butyl 2-[4-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]acetate;
methyl 2-[3-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]phenyl]acetate;
tert-butyl 2-[5-[2-[[6-[2,6-difluoro-3-[[(3R)-3-fluoropyrrolidin-1-yl]sulfonylamino]phenyl]-8-methyl-7-oxopyrido[2,3-d]pyrimidin-2-yl]amino]ethyl]pyrimidin-2-yl]acetate;
(3R)-N-[3-[2-[2-(2-acetyl-1,3-dihydroisoindol-5-yl)ethylamino]-8-methyl-7-oxopyrido[2,3-d]pyrimidin-6-yl]-2,4-difluorophenyl]-3-fluoropyrrolidine-1-sulfonamide; and
(3R)-N-[3-[2-[2-(2-acetyl-3,4-dihydro-1H-isoquinolin-7-yl)ethylamino]-8-methyl-7-oxopyrido[2,3-d]pyrimidin-6-yl]-2,4-difluorophenyl]-3-fluoropyrrolidine-1-sulfonamide;
or a pharmaceutically acceptable salt thereof.

12. A process for the preparation of a compound according to any one of claims 1 to 11 comprising one of the following steps:
(a) the reaction of a compound of formula (A1) 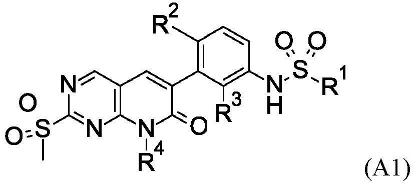
with a compound of formula (A2) 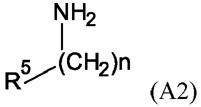
in the presence of a base; or
(b) the reaction of a compound of formula (B 1) 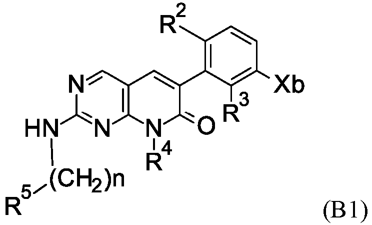
with a compound of formula (B2) 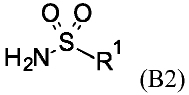
in the presence of a base;
wherein n, R¹, R², R³, R⁴ and R⁵ are as defined in any one of claims 1 to 10.

13. A compound according to any one of claims 1 to 11, for use as therapeutically active substance.

14. A pharmaceutical composition comprising a compound in accordance with any one of claims 1 to 11 and a therapeutically inert carrier.

15. A compound according to any one of claims 1 to 11 for use in the treatment or prophylaxis of cancer, in particular melanoma or NSCLC.

## Patentansprüche

1. Verbindung der Formel (I) 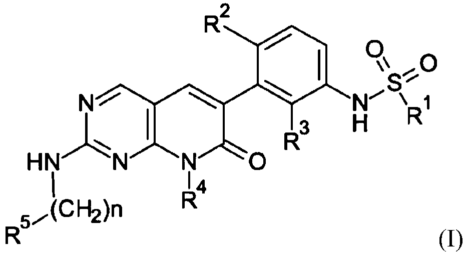 wobei
R¹ Dialkylamino, (Cycloalkyl)(alkyl)amino, Halogenheterocycloalkyl, Heterocycloalkyl oder Cycloalkyl ist;
R² und R³ unabhängig voneinander ausgewählt sind aus Wasserstoff, Cyano und Halogen;
R⁴ Alkyl, Alkoxycarbonylheterocycloalkyl, Aminoalkoxyalkoxyalkyl, Heterocycloalkyl, Alkylcarbonylaminoalkoxyalkoxyalkyl, Alkylcarbonylheterocycloalkyl, Alkylcarbonylaminocycloalkyl oder Phenylalkoxyalkyl ist;
n = 0, 1, 2, 3, 4 oder 5 ist; und
R⁵ Alkoxycarbonyl, Alkoxycarbonylalkylphenyl, Alkoxycarbonylalkylheterocycloalkyl, Alkoxycarbonylheterocycloalkyl, Alkylamino, Alkylcarbonyl(alkylamino), Alkylcarbonyl(alkylamino)alkylphenyl, Alkylcarbonylamino, Alkylcarbonylaminoalkoxy, Alkylcarbonylaminoalkoxyalkoxy, Alkylcarbonylheterocycloalkylaminocarbonyl, Alkylcarbonylaminoalkylphenyl, Alkylcarbonyl(hydroxyheterocycloalkyl), Alkylcarbonylheterocycloalkyl, Alkylcarbonylaminocycloalkyl, Aminoalkoxy, Cycloalkyl, Heterocycloalkyl, Heterocycloalkylaminocarbonyl, Phenyloxy, Hydroxyalkoxyalkoxy, Phenyl oder Wasserstoff ist;
oder ein pharmazeutisch verträgliches Salz davon.

2. Verbindung nach Anspruch 1, wobei R¹ Dialkylamino, (Cycloalkyl)(alkyl)amino, Halogenpyrrolidinyl, Pyrrolidinyl oder Cycloalkyl ist.

3. Verbindung nach Anspruch 1 oder 2, wobei R¹ (Ethyl)(methyl)amino, (Cyclopropyl)(methyl)amino, Fluorpyrrolidinyl, Pyrrolidinyl oder Cyclopentyl ist.

4. Verbindung nach einem der Ansprüche 1 bis 3, wobei R¹ Dialkylamino oder Halogenheterocycloalkyl ist.

5. Verbindung nach einem der Ansprüche 1 bis 4, wobei R² und R³ unabhängig voneinander ausgewählt sind aus Wasserstoff, Cyano und Fluor.

6. Verbindung nach einem der Ansprüche 1 bis 5, wobei R⁴ Methyl, tert-Butyloxycarbonylpyrrolidinyl, Aminoethoxyethoxyethyl, Pyrrolidinyl, Methylcarbonylaminoethoxyethoxyethyl, Methylcarbonylpyrrolidinyl, Methylcarbonylaminocyclobutyl oder Phenylmethoxyethyl ist.

7. Verbindung nach einem der Ansprüche 1 bis 6, wobei R⁴ Alkyl ist.

8. Verbindung nach einem der Ansprüche 1 bis 7, wobei n = 0, 1, 2, 3 oder 5.

9. Verbindung nach einem der Ansprüche 1 bis 8, wobei R⁵ tert-Butyloxycarbonyl, tert-Butyloxycarbonylmethylphenyl, Methoxycarbonylmethylphenyl, tert-Butyloxycarbonylpiperidinyl, tert-Butyloxycarbonylpiperazinyl, Methylamino, Methylcarbonyl(methylamino), Methylcarbonyl(methylamino)methylphenyl, Methylcarbonylamino, Methylcarbonylaminoethoxyethoxy, Methylcarbonylpiperidinylaminocarbonyl, Methylcarbonylaminomethylphenyl, Methylcarbonyl(hydroxypiperidinyl), Methylcarbonylpiperidinyl, Methylcarbonylpiperazinyl, Methylcarbonylpiperidinyl, Methylcarbonyl-1,3-dihydroisoindolyl, Methylcarbonyl-1H-isochinolinyl, Aminoethoxy, Cyclopropyl, Cyclopentyl, Piperidinyl, Piperidinylaminocarbonyl, Phenyloxy, Hydroxyethoxyethoxy, Phenyl oder Wasserstoff ist.

10. Verbindung nach einem der Ansprüche 1 bis 9, wobei R⁵ Phenyl, Alkoxycarbonyl, Cycloalkyl, Alkylcarbonylpiperidinyl, Alkylcarbonylpiperazinyl, Alkylcarbonylamino, Alkylcarbonyl(alkylamino), Alkylcarbonylaminoalkylphenyl oder Alkylcarbonyl(alkylamino)alkylphenyl ist.

11. Verbindung nach einem der Ansprüche 1 bis 10, ausgewählt aus
tert-Butyl-4-[2-[[6-[2,6-difluor-3-[[(3R)-3-fluorpyrrolidin-1-yl]sulfonylamino]phenyl]-8-methyl-7-oxopyrido[2,3-d]pyrimidin-2-yl]amino]ethyl]piperidin-1-carboxylat;
(3R)-N-[2,4-Difluor-3-[8-methyl-7-oxo-2-(2-phenylethylamino)pyrido[2,3-d]pyrimidin-6-yl]phenyl]-3-fluorpyrrolidin-1-sulfonamid;
tert-Butyl-4-[[6-[2,6-difluor-3-[[(3R)-3-fluorpyrrolidin-1-yl]sulfonylamino]phenyl]-8-methyl-7-oxopyrido[2,3-d]pyrimidin-2-yl]amino]butanoat;
(3R)-N-[2,4-Difluor-3-[2-[2-[2-(2-hydroxyethoxy)ethoxy]ethylamino]-8-methyl-7-oxopyrido[2,3-d]pyrimidin-6-yl]phenyl]-3-fluorpyrrolidin-1-sulfonamid;
(3R)-N-[2,4-Difluor-3-[8-methyl-7-oxo-2-(2-phenoxyethylamino)pyrido[2,3-d]pyrimidin-6-yl]phenyl]-3-fluorpyrrolidin-1-sulfonamid;
(3R)-N-[3-[2-(Cyclopentylamino)-8-methyl-7-oxopyrido[2,3-d]pyrimidin-6-yl]-2,4-difluorphenyl]-3-fluorpyrrolidin-1-sulfonamid;
tert-Butyl-4-[2-[[6-[2,6-difluor-3-(pyrrolidin-1-ylsulfonylamino)phenyl]-8-methyl-7-oxopyrido[2,3-d]pyrimidin-2-yl]amino]ethyl]piperidin-1-carboxylat;
6-[3-[[Ethyl(methyl)sulfamoyl]amino]-2,6-difluorphenyl]-2-[2-[2-(2-hydroxyethoxy)ethoxy]ethylamino]-8-methyl-7-oxopyrido[2,3-d]pyrimidin;
(3R)-N-[3-[2-(Cyclopropylmethylamino)-8-methyl-7-oxopyrido[2,3-d]pyrimidin-6-yl]-2,4-difluorphenyl]-3-fluorpyrrolidin-1-sulfonamid;
2-(Cyclopropylmethylamino)-6-[3-[[ethyl(methyl)sulfamoyl]amino]-2,6-difluorphenyl]-8-methyl-7-oxopyrido[2,3-d]pyrimidin;
2-(Cyclopropylmethylamino)-6-[3-[[cyclopropyl(methyl)sulfamoyl]amino]-2,6-difluorphenyl]-8-methyl-7-oxopyrido[2,3-d]pyrimidin;
tert-Butyl-4-[2-[[6-[3-(cyclopentylsulfonylamino)-2,6-difluorphenyl]-8-methyl-7-oxopyrido[2,3-d]pyrimidin-2-yl]amino]ethyl]piperidin-1-carboxylat;
(3R)-N-[2,4-Difluor-3-[8-methyl-7-oxo-2-(2-piperidin-4-ylethylamino)pyrido[2,3-d]pyrimidin-6-yl]phenyl]-3-fluorpyrrolidin-1-sulfonamid-hydrochlorid;
(3R)-N-[3-[2-[2-(2-Aminoethoxy)ethylamino]-8-methyl-7-oxopyrido[2,3-d]pyrimidin-6-yl]-2,4-difluorphenyl]-3-fluorpyrrolidin-1-sulfonamid-hydrochlorid;
(3R)-N-[2,4-Difluor-3-[8-methyl-2-[3-(methylamino)propylamino]-7-oxopyrido[2,3-d]-pyrimidin-6-yl]phenyl]-3-fluorpyrrolidin-1-sulfonamid-hydrochlorid;
(3R)-N-[2,4-Difluor-3-[8-methyl-7-oxo-2-(3-piperidin-4-ylpropylamino)pyrido[2,3-d]pyrimidin-6-yl]phenyl]-3-fluorpyrrolidin-1-sulfonamid-hydrochlorid;
(3R)-N-[3-[2-[2-(1-Acetylpiperidin-4-yl)ethylamino]-8-methyl-7-oxopyrido[2,3-d]pyrimidin-6-yl]-2,4-difluorphenyl]-3-fluorpyrrolidin-1-sulfonamid;
(3R)-N-[3-[2-[(1-Acetylpiperidin-4-yl)methylamino]-8-methyl-7-oxopyrido[2,3-d]pyrimidin-6-yl]-2,4-difluorphenyl]-3-fluorpyrrolidin-1-sulfonamid;
(3R)-N-[3-[2-[(1-Acetylazetidin-3-yl)amino]-8-methyl-7-oxopyrido[2,3-d]pyrimidin-6-yl]-2,4-difluorphenyl]-3-fluorpyrrolidin-1-sulfonamid;
N-[(1R,3R)-3-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]cyclopentyl]acetamid,
N-[(1S,3S)-3-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]cyclopentyl]acetamid;
N-[5-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]pentyl]acetamid,
(3R)-N-[3-[2-[2-(1-Acetyl-4-hydroxypiperidin-4-yl)ethylamino]-8-methyl-7-oxopyrido[2,3-d]pyrimidin-6-yl]-2,4-difluorphenyl]-3-fluorpyrrolidin-1-sulfonamid;
N-[3-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]propyl]-N-methylacetamid;
(3R)-N-[3-[2-[3-(1-Acetylpiperidin-4-yl)propylamino]-8-methyl-7-oxopyrido[2,3-d]pyrimidin-6-yl]-2,4-difluorphenyl]-3-fluorpyrrolidin-1-sulfonamid;
N-[2-[2-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]ethoxy]ethyl]acetamid;
N-[3-[[6-[3-[[Ethyl(methyl)sulfamoyl]amino]-2,6-difluorphenyl]-8-methyl-7-oxopyrido[2,3-d]pyrimidin-2-yl]amino]propyl]-N-methylacetamid;
(3R)-N-[3-[2-[2-(4-Acetylpiperazin-1-yl)ethylamino]-8-methyl-7-oxopyrido[2,3-d]-pyrimidin-6-yl]-2,4-difluorphenyl]-3-fluorpyrrolidin-1-sulfonamid;
N-[2-[2-[2-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]ethoxy]ethoxy]ethyl]acetamid,
N-[[3-[2-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]ethyl]phenyl]methyl]acetamid;
N-[[4-[2-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]ethyl]phenyl]methyl]acetamid;
N-[[3-[2-[[6-[2,6-Difluor-3-[[(3R)-3-fluorpyrrolidin-1-yl]sulfonylamino]phenyl]-8-methyl-7-oxopyrido[2,3-d]pyrimidin-2-yl]amino]ethyl]phenyl]methyl]-N-methylacetamid;
2-[2-(1-Acetylpiperidin-4-yl)ethylamino]-6-[2-cyano-3-[[ethyl(methyl)sulfamoyl]-amino]phenyl]-8-methyl-7-oxopyrido[2,3-d]pyrimidin;
tert-Butyl-(3RS)-3-[2-(cyclopropylmethylamino)-6-[2,6-difluor-3-[[(3R)-3-fluorpyrrolidin-1-yl]sulfonylamino]phenyl]-7-oxopyrido[2,3-d]pyrimidin-8-yl]pyrrolidin-1-carboxylat;
N-[2-[2-[2-[2-(Cyclopropylmethylamino)-6-[2,6-difluor-3-[[(3R)-3-fluorpyrrolidin-1-yl]sulfonylamino]phenyl]-7-oxopyrido[2,3-d]pyrimidin-8-yl]ethoxy]ethoxy]-ethyl]acetamid;
(3R)-N-[3-[8-((3RS)-1-Acetylpyrrolidin-3-yl)-2-(cyclopropylmethylamino)-7-oxopyrido[2,3-d]pyrimidin-6-yl]-2,4-difluorphenyl]-3-fluorpyrrolidin-1-sulfonamid, tert-Butyl-4-[2-[[6-[2,6-difluor-3-[[(3R)-3-fluorpyrrolidin-1-yl]sulfonylamino]phenyl]-8-methyl-7-oxopyrido[2,3-d]pyrimidin-2-yl]amino]ethyl]piperazin-1-carboxylat;
tert-Butyl-2-[4-[2-[[6-[2,6-difluor-3-[[(3R)-3-fluorpyrrolidin-1-yl]sulfonylamino]phenyl]-8-methyl-7-oxopyrido[2,3-d]pyrimidin-2-yl]amino]ethyl]phenyl]acetat;
Methyl-2-[3-[2-[[6-[2,6-difluor-3-[[(3R)-3-fluorpyrrolidin-1-yl]sulfonylamino]phenyl]-8-methyl-7-oxopyrido[2,3-d]pyrimidin-2-yl]amino]ethyl]phenyl]acetat;
tert-Butyl-2-[5-[2-[[6-[2,6-difluor-3-[[(3R)-3-fluorpyrrolidin-1-yl]sulfonylamino]phenyl]-8-methyl-7-oxopyrido[2,3-d]pyrimidin-2-yl]amino]ethyl]pyrimidin-2-yl]acetat;
(3R)-N-[3-[2-[2-(2-Acetyl-1,3-dihydroisoindol-5-yl)ethylamino]-8-methyl-7-oxopyrido[2,3-d]pyrimidin-6-yl]-2,4-difluorphenyl]-3-fluorpyrrolidin-1-sulfonamid; und
(3R)-N-[3-[2-[2-(2-Acetyl-3,4-dihydro-1H-isochinolin-7-yl)ethylamino]-8-methyl-7-oxopyrido[2,3-d]pyrimidin-6-yl]-2,4-difluorphenyl]-3-fluorpyrrolidin-1-sulfonamid;
oder ein pharmazeutisch verträgliches Salz davon.

12. Verfahren zur Herstellung einer Verbindung nach einem der Ansprüche 1 bis 11, umfassend einen der folgenden Schritte:
(a) die Reaktion einer Verbindung der Formel (A1) 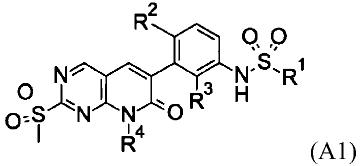
mit einer Verbindung der Formel (A2) 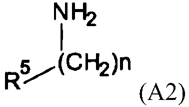 in Gegenwart einer Base; oder
(b) die Reaktion einer Verbindung der Formel (B1) 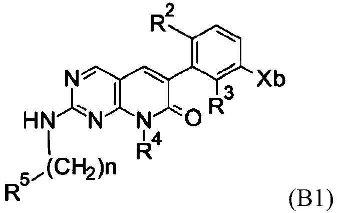
mit einer Verbindung der Formel (B2) 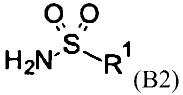
in Gegenwart einer Base;
wobei n, R¹, R², R³, R⁴ und R⁵ wie in einem der Ansprüche 1 bis 10 definiert sind.

13. Verbindung nach einem der Ansprüche 1 bis 11 zur Verwendung als therapeutisch wirksame Substanz.

14. Pharmazeutische Zusammensetzung, umfassend eine Verbindung nach einem der Ansprüche 1 bis 11 und einen therapeutisch inerten Träger.

15. Verbindung nach einem der Ansprüche 1 bis 11 zur Verwendung bei der Behandlung oder Prophylaxe von Krebs, insbesondere Melanom oder NSCLC.

## Revendications

1. Composé de formule (I) 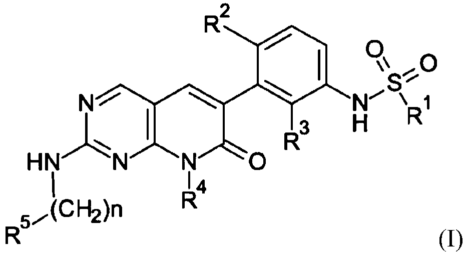 dans lequel
R¹ représente un dialkylamino, un (cycloalkyl)(alkyl)amino, un halogénohétérocycloalkyle, un hétérocycloalkyle ou un cycloalkyle ;
R² et R³ sont indépendamment choisis parmi un hydrogène, un cyano et un halogène ;
R⁴ représente un alkyle, un alcoxycarbonylhétérocycloalkyle, un aminoalcoxyalcoxyalkyle, un hétérocycloalkyle, un alkylcarbonylaminoalcoxyalcoxyalkyle, un alkylcarbonylhétérocycloalkyle, un alkylcarbonylaminocycloalkyle ou un phénylalcoxyalkyle ;
n = 0, 1, 2, 3, 4 ou 5 ; et
R⁵ représente un alcoxycarbonyle, un alcoxycarbonylalkylphényle, un alcoxycarbonylalkylhétérocycloalkyle, un alcoxycarbonylhétérocycloalkyle, un alkylamino, un alkylcarbonyl(alkylamino), un alkylcarbonyl(alkylamino)alkylphényle, un alkylcarbonylamino, un alkylcarbonylaminoalcoxy, un alkylcarbonylaminoalcoxyalcoxy, un alkylcarbonylhétérocycloalkylaminocarbonyle, un alkylcarbonylaminoalkylphényle, un alkylcarbonyl(hydroxyhétérocycloalkyle), un alkylcarbonylhétérocycloalkyle, un alkylcarbonylaminocycloalkyle, un aminoalcoxy, un cycloalkyle, un hétérocycloalkyle, un hétérocycloalkylaminocarbonyle, un phényloxy, un hydroxyalcoxyalcoxy, un phényle ou un hydrogène ;
ou un sel pharmaceutiquement acceptable de celui-ci.

2. Composé selon la revendication 1, dans lequel R¹ représente un dialkylamino, un (cycloalkyl)(alkyl)amino, un halogénopyrrolidinyle, un pyrrolidinyle ou un cycloalkyle.

3. Composé selon la revendication 1 ou 2, dans lequel R¹ représente un
(éthyl)(méthyl)amino, un (cyclopropyl)(méthyl)amino, un fluoropyrrolidinyle, un pyrrolidinyle ou un cyclopentyle.

4. Composé selon l'une quelconque des revendications 1 à 3, dans lequel R¹ représente un dialkylamino ou un halogénohétérocycloalkyle.

5. Composé selon l'une quelconque des revendications 1 à 4, dans lequel R² et R³ sont indépendamment choisis parmi un hydrogène, un cyano et un fluor.

6. Composé selon l'une quelconque des revendications 1 à 5, dans lequel R⁴ représente un méthyle, un tert-butyloxycarbonylpyrrolidinyle, un aminoéthoxyéthoxyéthyle, un pyrrolidinyle, un méthylcarbonylaminoéthoxyéthoxyéthyle, un méthylcarbonylpyrrolidinyle, un méthylcarbonylaminocyclobutyle ou un phénylméthoxyéthyle.

7. Composé selon l'une quelconque des revendications 1 à 6, dans lequel R⁴ représente un alkyle.

8. Composé selon l'une quelconque des revendications 1 à 7, dans lequel n = 0, 1, 2, 3 ou 5.

9. Composé selon l'une quelconque des revendications 1 à 8, dans lequel R⁵ représente un tert-butyloxycarbonyle, un tert-butyloxycarbonylméthylphényle, un méthoxycarbonylméthylphényle, un tert-butyloxycarbonylpipéridinyle, un tert-butyloxycarbonylpipérazinyle, un méthylamino, un méthylcarbonyl(méthylamino), un méthylcarbonyl(méthylamino)méthylphényle, un méthylcarbonylamino, un méthylcarbonylaminoéthoxyéthoxy, un méthylcarbonylpipéridinylaminocarbonyle, un méthylcarbonylaminométhylphényle, un méthylcarbonyl(hydroxypipéridinyle), un méthylcarbonylpipéridinyle, un méthylcarbonylpipérazinyle, un méthylcarbonylpipéridinyle, un méthylcarbonyl-1,3-dihydroisoindolyle, un méthylcarbonyl-1H-isoquinoléinyle, un aminoéthoxy, un cyclopropyle, un cyclopentyle, un pipéridinyle, un pipéridinylaminocarbonyle, un phényloxy, un hydroxyéthoxyéthoxy, un phényle ou un hydrogène.

10. Composé selon l'une quelconque des revendications 1 à 9, dans lequel R⁵ représente un phényle, un alcoxycarbonyle, un cycloalkyle, un alkylcarbonylpipéridinyle, un alkylcarbonylpipérazinyle, un alkylcarbonylamino, un alkylcarbonyl(alkylamino), un alkylcarbonylaminoalkylphényle ou un alkylcarbonyl(alkylamino)alkylphényle.

11. Composé selon l'une quelconque des revendications 1 à 10 choisi parmi
le 4-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]pipéridine-1-carboxylate de tert-butyle ;
le (3R)-N-[2,4-difluoro-3-[8-méthyl-7-oxo-2-(2-phényléthylamino)pyrido[2,3-d]pyrimidin-6-yl]phényl]-3-fluoropyrrolidine-1-sulfonamide ;
le 4-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]butanoate de tert-butyle ;
le (3R)-N-[2,4-difluoro-3-[2-[2-[2-(2-hydroxyéthoxy)éthoxy]éthylamino]-8-méthyl-7-oxopyrido[2,3-d]pyrimidin-6-yl]phényl]-3-fluoropyrrolidine-1-sulfonamide ;
le (3R)-N-[2,4-difluoro-3-[8-méthyl-7-oxo-2-(2-phénoxyéthylamino)pyrido[2,3-d]pyrimidin-6-yl]phényl]-3-fluoropyrrolidine-1-sulfonamide ;
le (3R)-N-[3-[2-(cyclopentylamino)-8-méthyl-7-oxopyrido[2,3-d]pyrimidin-6-yl]-2,4-difluorophényl]-3-fluoropyrrolidine-1-sulfonamide ;
le 4-[2-[[6-[2,6-difluoro-3-(pyrrolidin-1-ylsulfonylamino)phényl]-8-méthyl-7-oxopyrido[2,3-d]pyrimidin-2-yl]amino]éthyl]pipéridine-1-carboxylate de tert-butyle ;
la 6-[3-[[éthyl(méthyl)sulfamoyl]amino]-2,6-difluorophényl]-2-[2-[2-(2-hydroxyéthoxy)éthoxy]éthylamino]-8-méthyl-7-oxopyrido[2,3-d]pyrimidine ;
le (3R)-N-[3-[2-(cyclopropylméthylamino)-8-méthyl-7-oxopyrido[2,3-d]pyrimidin-6-yl]-2,4-difluorophényl]-3-fluoropyrrolidine-1-sulfonamide ;
la 2-(cyclopropylméthylamino)-6-[3-[[éthyl(méthyl)sulfamoyl]amino]-2,6-difluorophényl]-8-méthyl-7-oxopyrido[2,3-d]pyrimidine ;
la 2-(cyclopropylméthylamino)-6-[3-[[cyclopropyl(méthyl)sulfamoyl]amino]-2,6-difluorophényl]-8-méthyl-7-oxopyrido[2,3-d]pyrimidine ;
le 4-[2-[[6-[3-(cyclopentylsulfonylamino)-2,6-difluorophényl]-8-méthyl-7-oxopyrido[2,3-d]pyrimidin-2-yl]amino]éthyl]pipéridine-1-carboxylate de tert-butyle ;
le chlorhydrate de (3R)-N-[2,4-difluoro-3-[8-méthyl-7-oxo-2-(2-pipéridin-4-yléthylamino)pyrido[2,3-d]pyrimidin-6-yl]phényl]-3-fluoropyrrolidine-1-sulfonamide ;
le chlorhydrate de (3R)-N-[3-[2-[2-(2-aminoéthoxy)éthylamino]-8-méthyl-7-oxopyrido[2,3-d]pyrimidin-6-yl]-2,4-difluorophényl]-3-fluoropyrrolidine-1-sulfonamide ;
le chlorhydrate de (3R)-N-[2,4-difluoro-3-[8-méthyl-2-[3-(méthylamino)propylamino]-7-oxopyrido[2,3-d]pyrimidin-6-yl]phényl]-3-fluoropyrrolidine-1-sulfonamide ;
le chlorhydrate de (3R)-N-[2,4-difluoro-3-[8-méthyl-7-oxo-2-(3-pipéridin-4-ylpropylamino)pyrido[2,3-d]pyrimidin-6-yl]phényl]-3-fluoropyrrolidine-1-sulfonamide ;
le (3R)-N-[3-[2-[2-(1-acétylpipéridin-4-yl)éthylamino]-8-méthyl-7-oxopyrido[2,3-d]pyrimidin-6-yl]-2,4-difluorophényl]-3-fluoropyrrolidine-1-sulfonamide ;
le (3R)-N-[3-[2-[(1-acétylpipéridin-4-yl)méthylamino]-8-méthyl-7-oxopyrido[2,3-d]pyrimidin-6-yl]-2,4-difluorophényl]-3-fluoropyrrolidine-1-sulfonamide ;
le (3R)-N-[3-[2-[(1-acétylazétidin-3-yl)amino]-8-méthyl-7-oxopyrido[2,3-d]pyrimidin-6-yl]-2,4-difluorophényl]-3-fluoropyrrolidine-1-sulfonamide ;
le N-[(1R,3R)-3-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]cyclopentyl]acétamide ;
le N-[(1S,3S)-3-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]cyclopentyl]acétamide ;
le N-[5-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]pentyl]acétamide ;
le (3R)-N-[3-[2-[2-(1-acétyl-4-hydroxypipéridin-4-yl)éthylamino]-8-méthyl-7-oxopyrido[2,3-d]pyrimidin-6-yl]-2,4-difluorophényl]-3-fluoropyrrolidine-1-sulfonamide ;
le N-[3-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]propyl]-N-méthylacétamide ;
le (3R)-N-[3-[2-[3-(1-acétylpipéridin-4-yl)propylamino]-8-méthyl-7-oxopyrido[2,3-d]pyrimidin-6-yl]-2,4-difluorophényl]-3-fluoropyrrolidine-1-sulfonamide ;
le N-[2-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthoxy]éthyl]acétamide ;
le N-[3-[[6-[3-[[éthyl(méthyl)sulfamoyl]amino]-2,6-difluorophényl]-8-méthyl-7-oxopyrido[2,3-d]pyrimidin-2-yl]amino]propyl]-N-méthylacétamide ;
le (3R)-N-[3-[2-[2-(4-acétylpipérazin-1-yl)éthylamino]-8-méthyl-7-oxopyrido[2,3-d]pyrimidin-6-yl]-2,4-difluorophényl]-3-fluoropyrrolidine-1-sulfonamide ;
le N-[2-[2-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthoxy]éthoxy]éthyl]acétamide ;
le N-[[3-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]phényl]méthyl]acétamide ;
le N-[[4-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]phényl]méthyl]acétamide ;
le N-[[3-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]phényl]méthyl]-N-méthylacétamide ;
la 2-[2-(1-acétylpipéridin-4-yl)éthylamino]-6-[2-cyano-3-[[éthyl(méthyl)sulfamoyl]amino]phényl]-8-méthyl-7-oxopyrido[2,3-d]pyrimidine ;
le (3RS)-3-[2-(cyclopropylméthylamino)-6-[2,6-difluoro-3-[[(3R)-3-fluoropyrrolidin-1-yl]sulfonylamino]phényl]-7-oxopyrido[2,3-d]pyrimidin-8-yl]pyrrolidine-1-carboxylate de tert-butyle ;
le N-[2-[2-[2-[2-(cyclopropylméthylamino)-6-[2,6-difluoro-3-[[(3R)-3-fluoropyrrolidin-1-yl]sulfonylamino]phényl]-7-oxopyrido[2,3-d]pyrimidin-8-yl]éthoxy]éthoxy]éthyl]acétamide ;
le (3R)-N-[3-[8-((3RS)-1-acétylpyrrolidin-3-yl)-2-(cyclopropylméthylamino)-7-oxopyrido[2,3-d]pyrimidin-6-yl]-2,4-difluorophényl]-3-fluoropyrrolidine-1-sulfonamide ;
le 4-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]pipérazine-1-carboxylate de tert-butyle ;
le 2-[4-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]phényl]acétate de tert-butyle ;
le 2-[3-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]phényl]acétate de méthyle ;
le 2-[5-[2-[[6-[2,6-difluoro-3-[[(3R)-3-fluoropyrrolidin-1-yl]sulfonylamino]phényl]-8-méthyl-7-oxopyrido[2,3-d]pyrimidin-2-yl]amino]éthyl]pyrimidin-2-yl]acétate de tert-butyle ;
le (3R)-N-[3-[2-[2-(2-acétyl-1,3-dihydroisoindol-5-yl)éthylamino]-8-méthyl-7-oxopyrido[2,3-d]pyrimidin-6-yl]-2,4-difluorophényl]-3-fluoropyrrolidine-1-sulfonamide ; et
le (3R)-N-[3-[2-[2-(2-acétyl-3,4-dihydro-1H-isoquinoléin-7-yl)éthylamino]-8-méthyl-7-oxopyrido[2,3-d]pyrimidin-6-yl]-2,4-difluorophényl]-3-fluoropyrrolidine-1-sulfonamide ;
ou un sel pharmaceutiquement acceptable de ceux-ci.

12. Procédé de préparation d'un composé selon l'une quelconque des revendications 1 à 11 comprenant l'une des étapes suivantes :
(a) la réaction d'un composé de formule (A1) 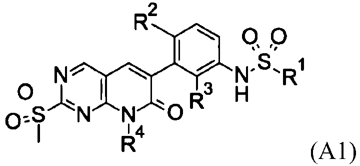
avec un composé de formule (A2) 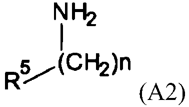
en présence d'une base ; ou
(b) la réaction d'un composé de formule (B1) 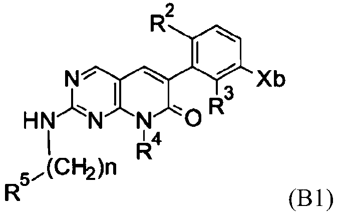
avec un composé de formule (B2) 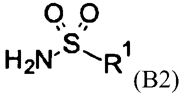
en présence d'une base ;
dans lequel n, R¹, R², R³, R⁴ et R⁵ sont tels que définis dans l'une quelconque des revendications 1 à 10.

13. Composé selon l'une quelconque des revendications 1 à 11, pour une utilisation comme substance thérapeutiquement active.

14. Composition pharmaceutique comprenant un composé selon l'une quelconque des revendications 1 à 11 et un véhicule thérapeutiquement inerte.

15. Composé selon l'une quelconque des revendications 1 à 11 pour une utilisation dans le traitement ou la prophylaxie du cancer, en particulier du mélanome ou du CPNPC.
